# EUROPEAN PATENT APPLICATION

(11) **EP 4 444 066 A1**
(43) Date of publication of application: **09.10.2024**
(21) Application number: 23206890.8
(22) Date of filing: 31.10.2023
(51) Int. Cl.: H10K 85/60, H10K 50/15, H10K 50/17

(54) **ORGANIC ELECTROLUMINESCENT DEVICE**

(30) Priority: 07.04.2023 CN 202310371469
(71) Applicant: NANJING TOPTO SEMICONDUCTOR MATERIAL CO., LTD., Nanjing Jiangsu 210000 (CN)
(72) Inventor: XU, Jun, NANJING, 210000 (CN); HUANG, Minghui, NANJING, 210000 (CN); MA, Rujie, NANJING, 210000 (CN)
(74) Representative: Hautier IP - MC/EP

(57) **Abstract**

The present disclosure discloses an organic electroluminescent device. The device of the present disclosure has at least one layer in the hole transport layer that is a compound with an alkyl, cycloalkyl, deuterated alkyl, or deuterated cycloalkyl substitution on the biphenyl group; and substitution of alkyl, cycloalkyl, deuterated alkyl, deuterated cycloalkyl has the following advantages: larger spatial steric hindrance and torque, higher hole mobility, better solubility, higher efficiency and longer lifespan, and higher lateral resistance. Therefore, when paired with the P-dopant material currently used in the market, the lateral leakage of the device is significantly reduced and the technical problem of client-side crosstalk is solved.

## Description

### Technical Field

The present disclosure relates to the field of organic electroluminescence technology, and specifically relates to an organic electroluminescent device.

### Background Art

OLED device consists of substrate, cathode, anode, hole injection layer (HIL), electron injection layer (EIL), hole transport layer (HTL), electron transport layer (ETL), electron barrier layer (EBL), hole barrier layer (HBL), emissive layer (EML) and other parts. When the two electrodes of the OLED device are applied with a voltage, positive and negative charges are generated in the functional material film layer of the organic layer through the action of electric field, and positive and negative charges are further compounded in the emissive layer, so that the light can be produced.

At present, the structure of OLED device generally consists of substrate, cathode, anode, hole injection layer (HIL), electron injection layer (EIL), hole transport layer (HTL), electron transport layer (ETL), electron barrier layer (EBL), hole barrier layer (HBL), and emissive layer (EML).

Current research on OLED devices to improve performance includes: improving the device structure, reducing the driving voltage of the device, improving the luminous efficiency of the device, improving the service life of the device and so on. In order to realize the continuous improvement of the performance of organic electroluminescent devices, it not only requires the innovation of the structure and the production process of organic electroluminescent devices, but also requires the continuous research and innovation of organic electroluminescent functional materials, so as to create organic electroluminescent functional materials with higher performance.

### Summary

The object of the present disclosure is that in response to the above technical problems, the present disclosure provides an organic electroluminescent device.

In order to achieve the above object of the present disclosure, the technical solutions adopted in the present disclosure are as follows.

An organic electroluminescent device includes a cathode, an anode, and an organic layer formed between the cathode and the anode, wherein the organic layer contains an emissive layer.

A hole transport region is provided between the anode and the emissive layer, wherein the hole transport region comprises a hole transport layer, or further comprises at least one of a hole injection layer and an electron blocking layer.

What between the emissive layer and the cathode comprises an electron transport region selected from at least one of an electron transport layer, a hole blocking layer, and an electron injection layer.

At least one layer of the hole transport region contains an organic electroluminescent compound as shown in formula 1 below, 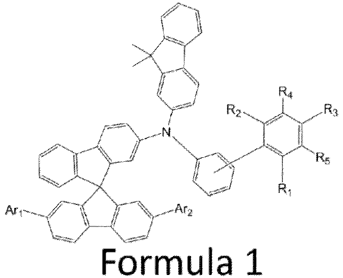 wherein
R₁-R₅ are each independently hydrogen, deuterium, deuterated, or undeuterated alkyl, or deuterated or undeuterated cycloalkyl, and R₁-R₅ are not hydrogen at the same time or R₁-R₃ are not hydrogen at the same time; and
Ar, and Ar₂ are each independently hydrogen or a C1-C4 alkyl.

Preferably, R₁-R₅ are each independently hydrogen, a deuterated or undeuterated alkyl of C1-C4, a deuterated or undeuterated cycloalkyl of C3-C6, and R₁-R₅ are not hydrogen at the same time.

Further preferably, R₁-R₅ are each independently hydrogen, deuterated or undeuterated methyl, deuterated or undeuterated cyclobutyl, deuterated or undeuterated cyclopentyl or deuterated or undeuterated cyclohexyl.

Preferably, Ar₁ and Ar₂ are each independently hydrogen or tert-butyl.

Preferably, the at least one layer in the hole transport region contains one or more of the compounds shown below:

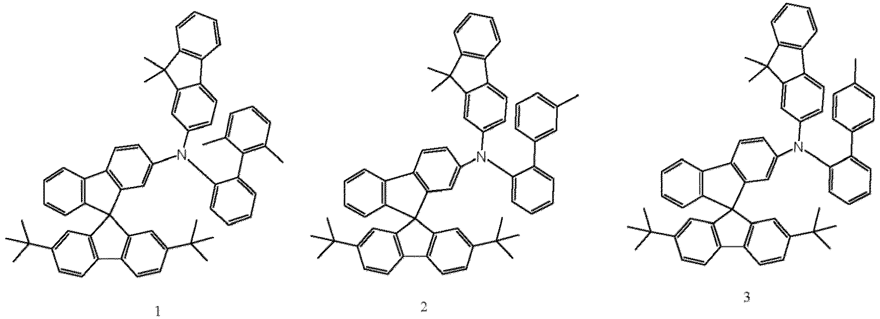

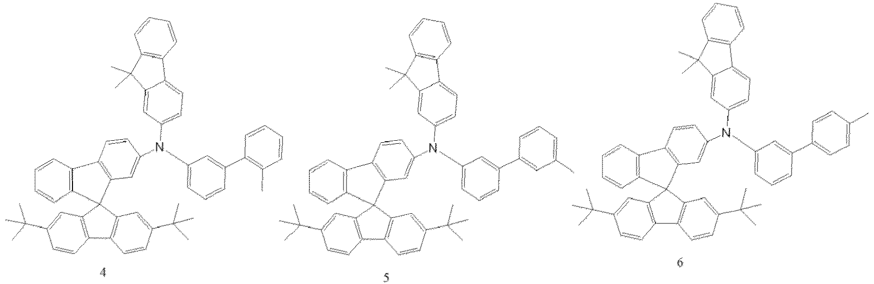

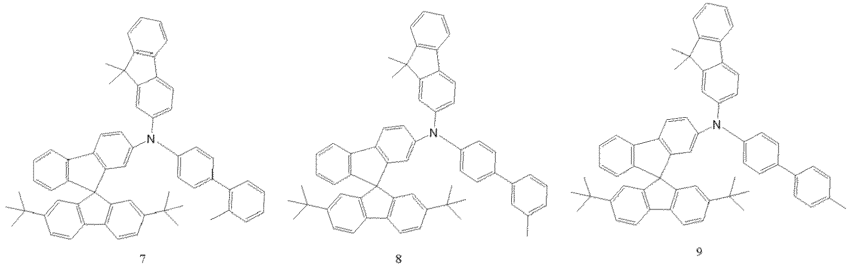

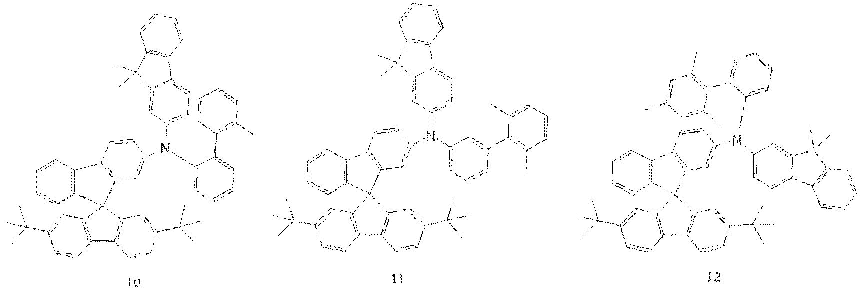

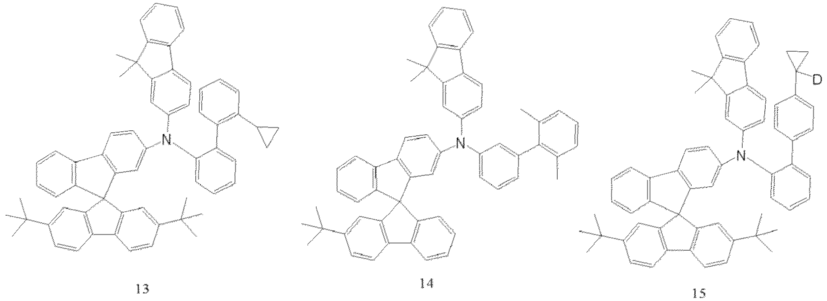

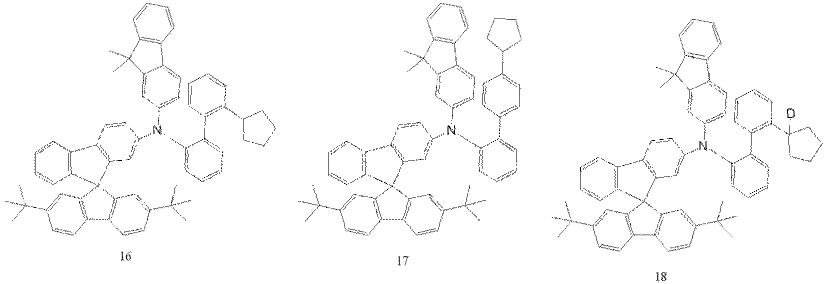

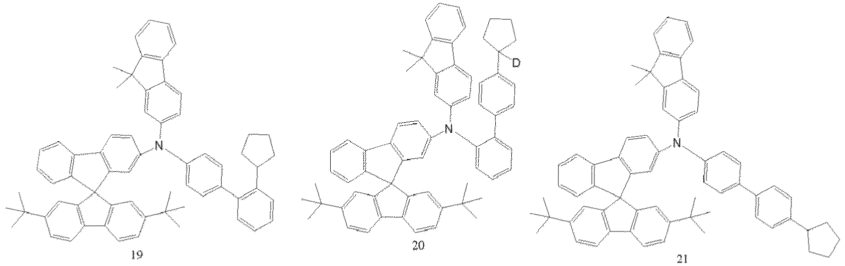

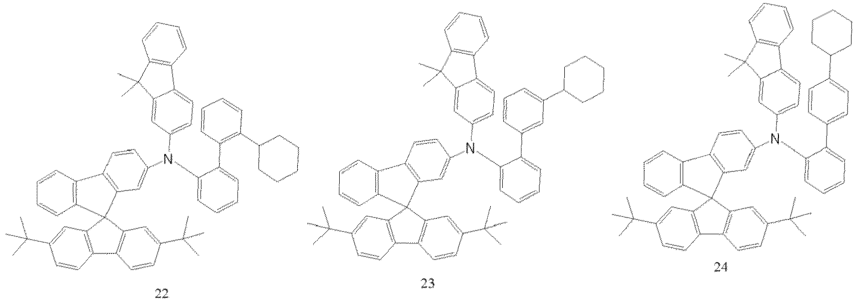

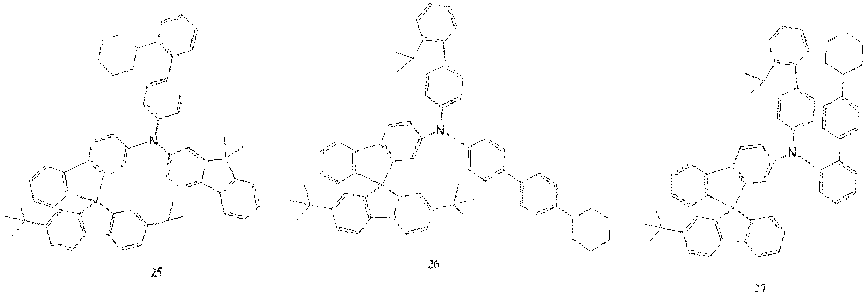

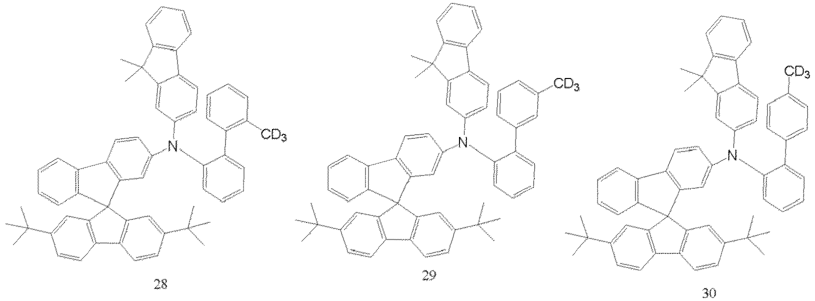

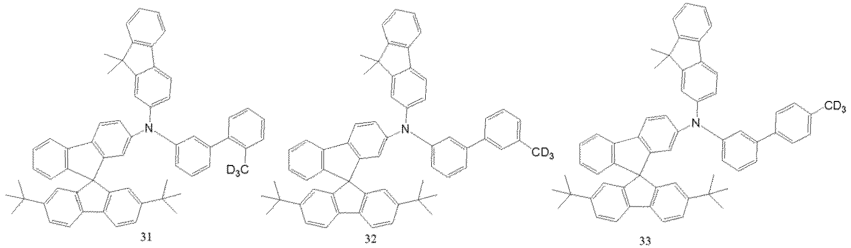

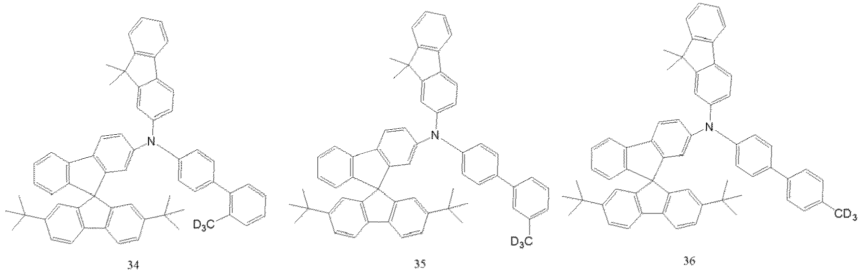

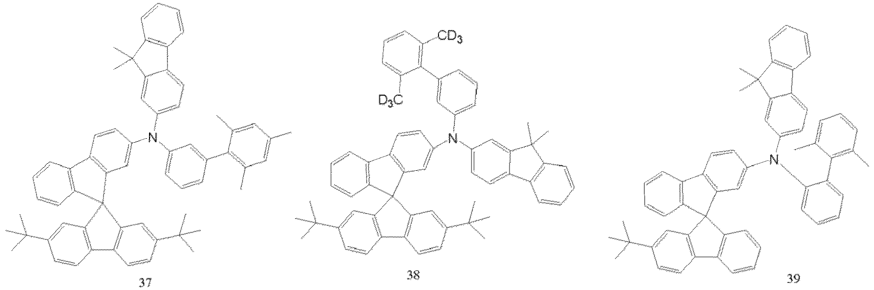

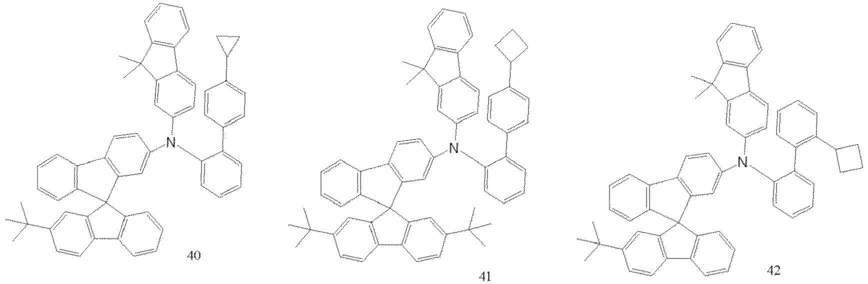

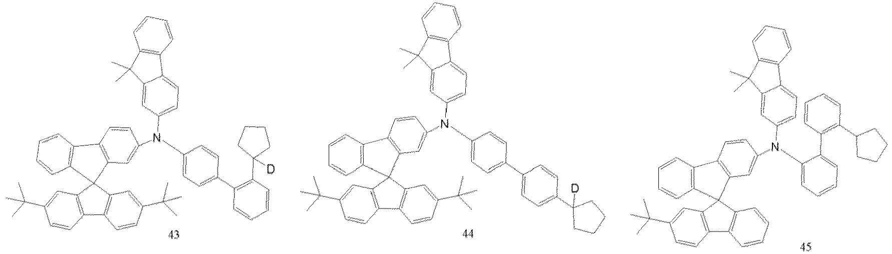

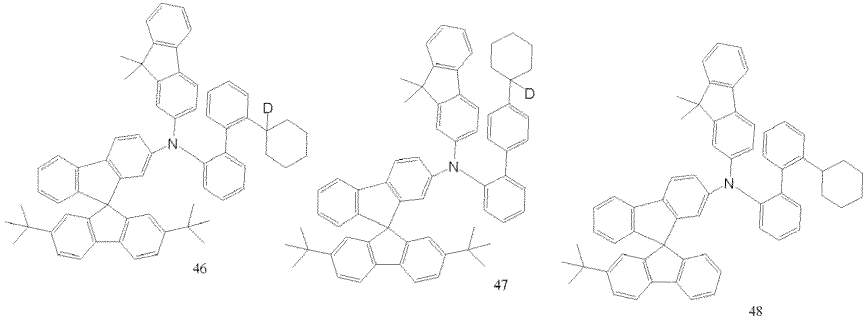

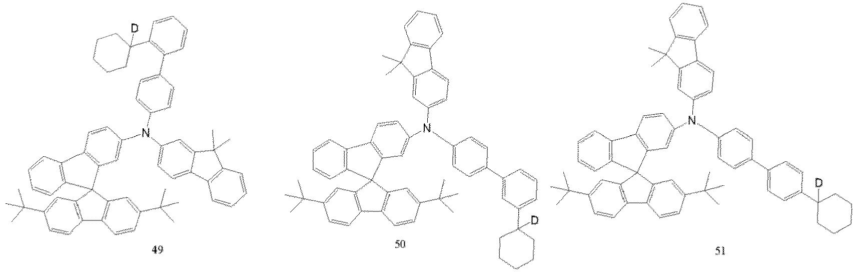

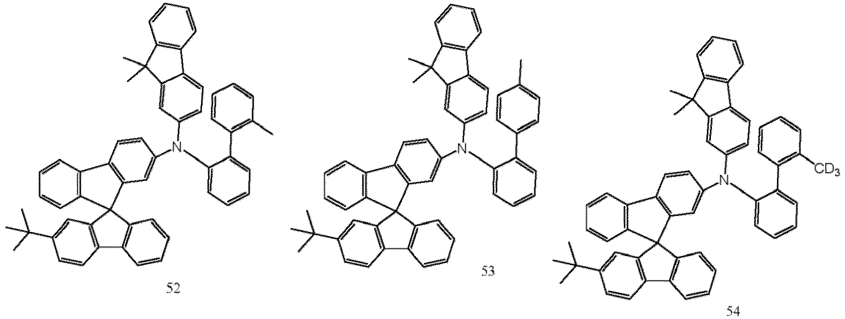

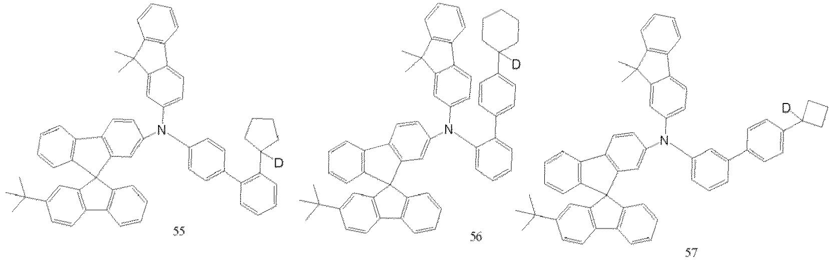

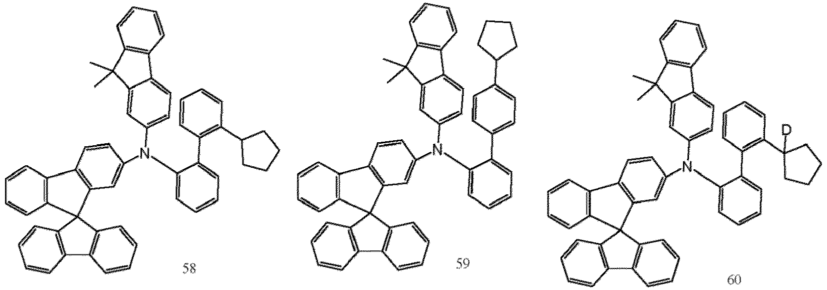

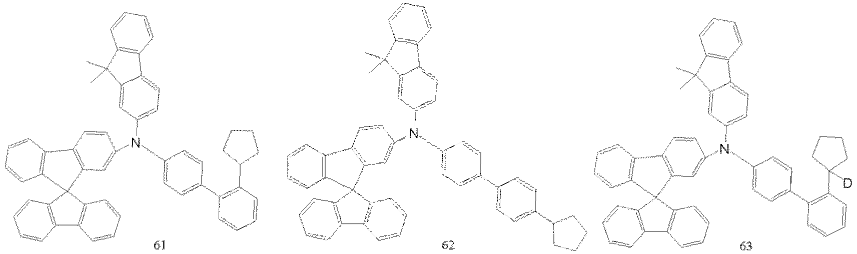

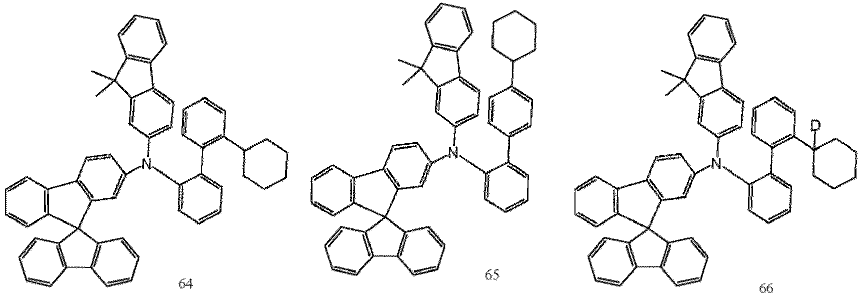

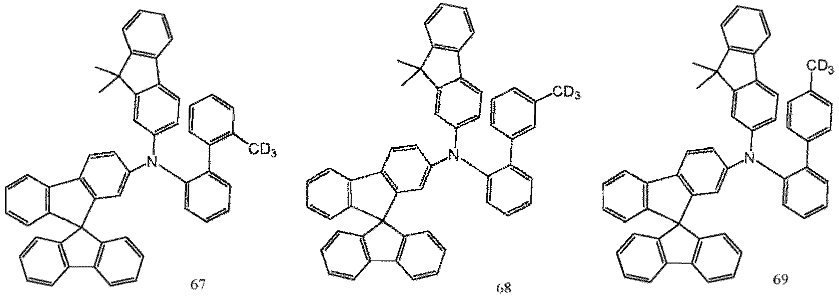

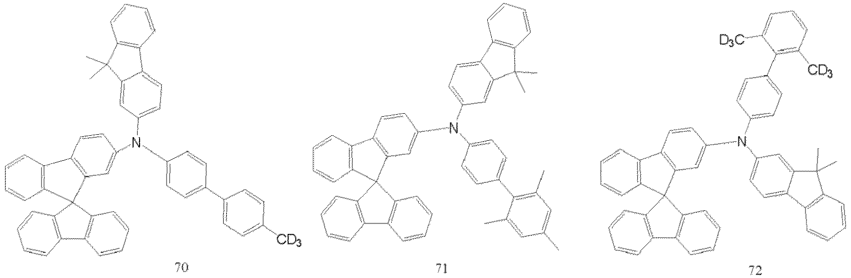

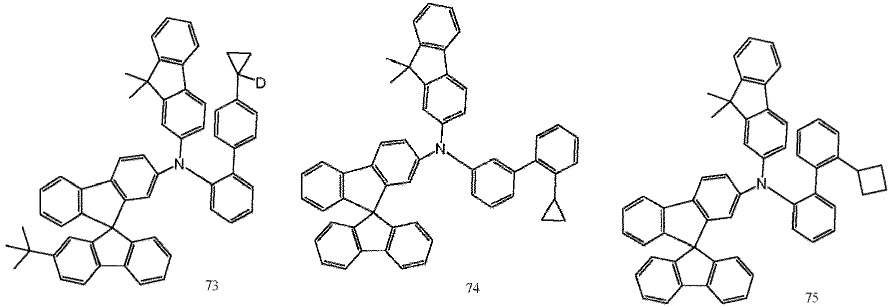

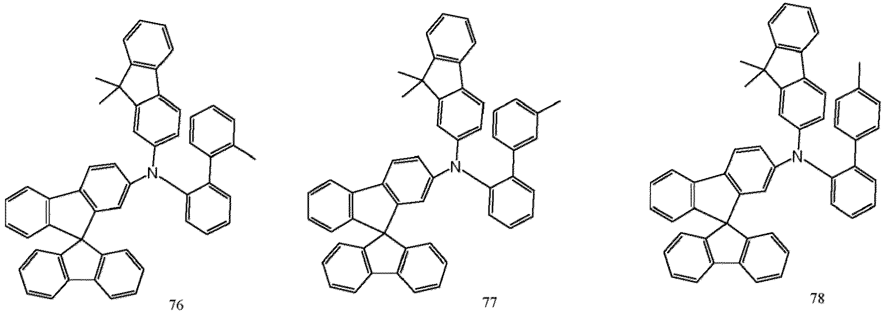

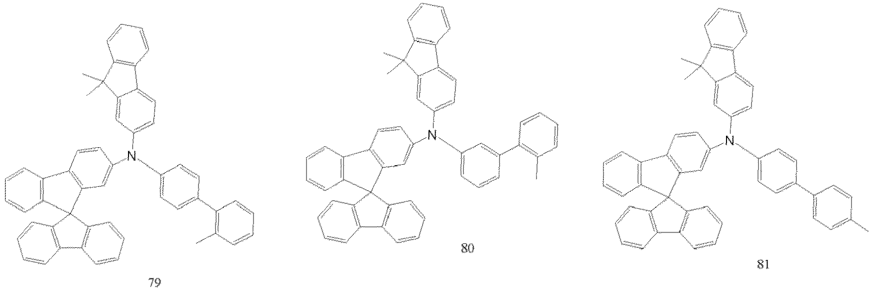

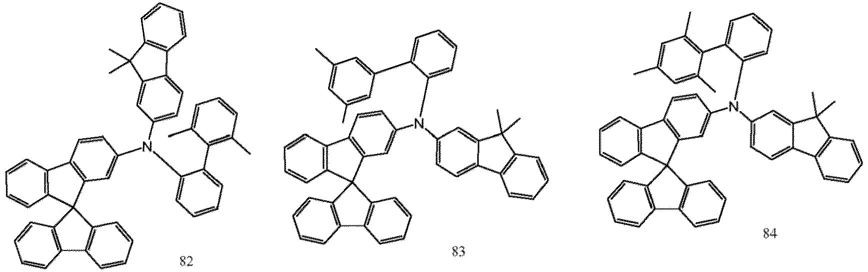

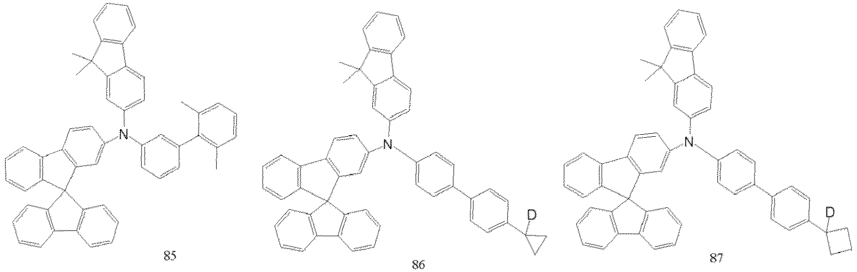

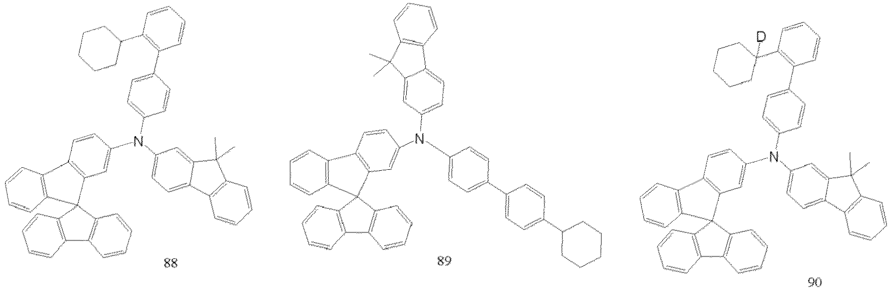

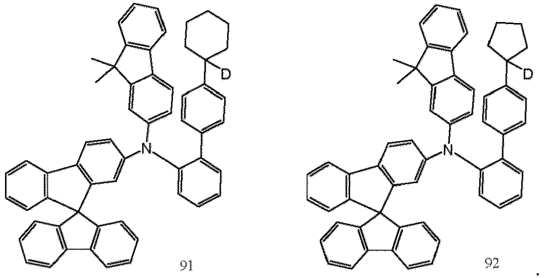

Further, a compound as shown in formula A can be contained in the hole injection layer:

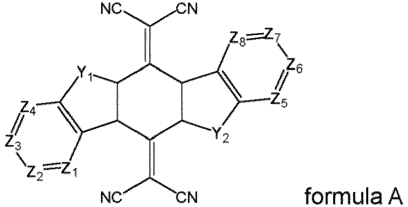

In formula A, Y₁ and Y₂ are the same or different, and are independent of each other as O or S;
Z₁-Z₈ are each independently N, CH or CR₄; and
R₄ is an alkyl of C1-C4, and at least one hydrogen in the alkyl of C1-C4 is substituted or unsubstituted with a deuterium atom or a fluorine atom;
or the hole injection layer contains compounds as shown in formula B: 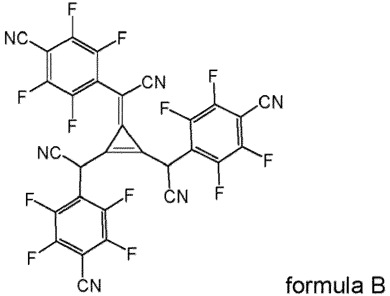
or the hole injection layer contains compounds as shown in formula C: 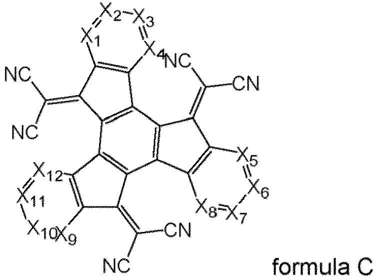
in formula C, X₁-X₁₂ are each independently selected from CR₅ or N;
R₅ selects from the group consisting of: hydrogen, deuterium, halogen, cyano, substituted or unsubstituted alkyl with 1-20 carbon atoms, substituted or unsubstituted cycloalkyl with 3-20 ring carbon atoms, substituted or unsubstituted heteroalkyl having 1-20 carbon atoms, substituted or unsubstituted arylalkyl with 7-30 carbon atoms, substituted or unsubstituted alkoxy with 1-20 carbon atoms, substituted or unsubstituted aryloxy with 6-30 carbon atoms, substituted or unsubstituted alkenyl with 2-20 carbon atoms, substituted or unsubstituted aryl with 6-30 carbon atoms, substituted or unsubstituted heteroaryl with 3-30 carbon atoms, substituted or unsubstituted alkylsilyl with 3-20 carbon atoms, substituted or unsubstituted arylsilyl with 6-20 carbon atoms, and amine, acyl, carbonyl, carboxylic, ester, nitrile, isonitrile, thio, sulfinyl, sulfonyl, and phosphonyl substituted or unsubstituted with 0-20 carbon atoms, and combinations thereof.

When more than one R₅ groups are present, R₅ is the same or different; and
any adjacent R₅ groups can optionally be linked to form a ring or a fused structure.

Preferably, R₄ is methyl or trifluoromethyl.

Preferably, Y₁ and Y₂ are identical, and are O or S.

Preferably, Z₁, Z₂, Z₇ or Z₈ is CH or CR₄, Z₄ or Z₅ is N or CH, and Z₃ or Z₆ is CR₄.

Preferably, in the compound shown in formula A, Z₁, Z₂, Z₇ or Z₈ is CH or CR₄, Z₄ or Z₅ is N or CH, and Z₃ or Z₆ is CR₄.

Further preferably, the compounds shown in formula A is any one of the following:

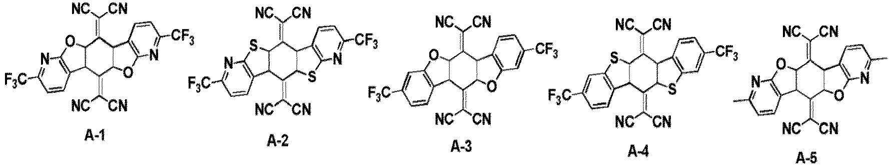

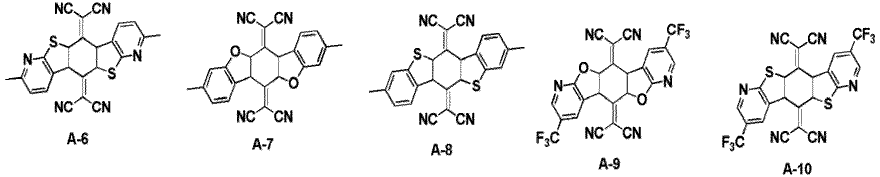

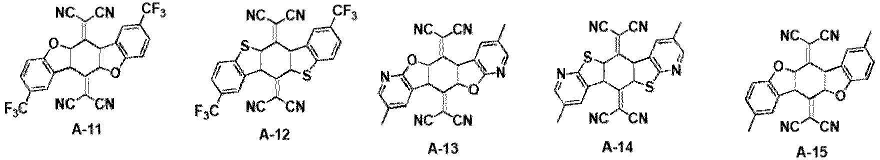

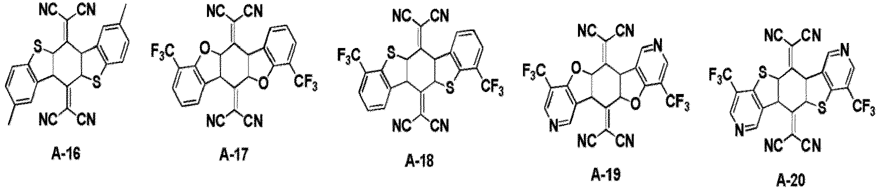

Preferably, in the compounds shown in formula C, X₁-X₁₂ are each independently selected from CR₅ or N, R₅ is one or more of hydrogen, halogen, cyano, alkyl of C1-C4, haloalkyl of C1-C4, alkoxy of C1-C4, haloalkoxy of C1-C4 or halothio.

More preferably, in the compounds shown in formula C, X₁-X₁₂ are each independently selected from CR₅ or N, R₅ is one or more of hydrogen, halogen, cyano, methyl, trifluoromethyl, methoxy, halomethoxy, or sulfur pentafluoride group.

Further preferably, the compounds shown in formula C is any one of the following:

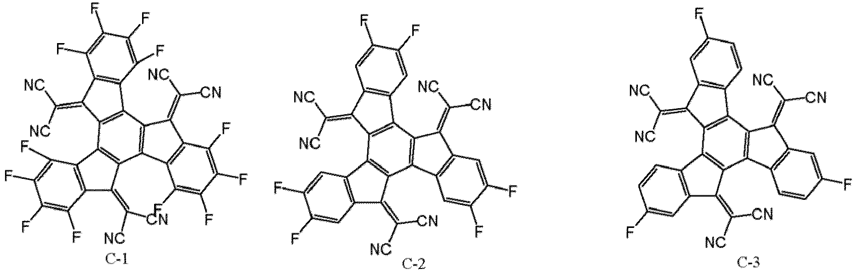

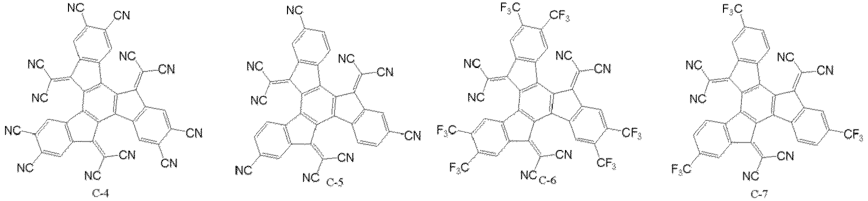

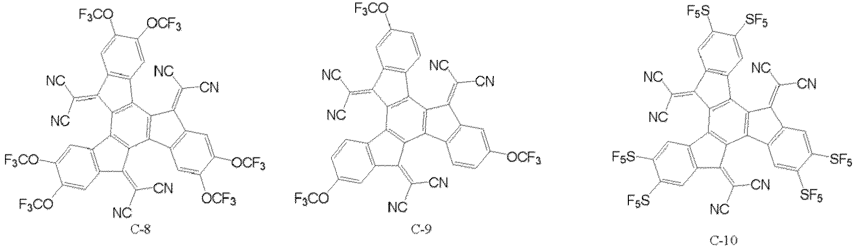

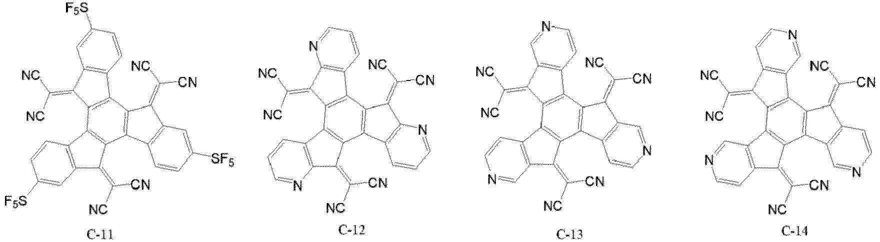

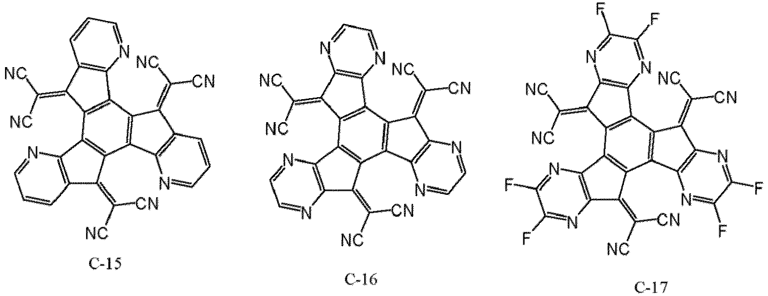

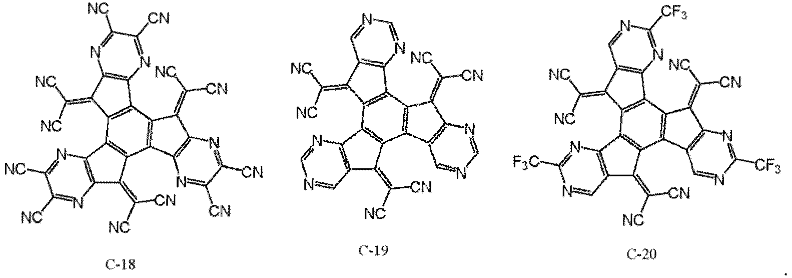

All room temperatures described in the present disclosure are 25±5 °C.

Beneficial effects of the present disclosure are as follow.

The present disclosure designs a new type of organic electroluminescent devices. This type of devices has characteristics as follows.

At least one layer in the hole transport region of the devices of the present disclosure is a compound with an alkyl, cycloalkyl, deuterated alkyl, or deuterated cycloalkyl substitution on the biphenyl group. The substitution of alkyl, cycloalkyl, deuterated alkyl, deuterated cycloalkyl has the following advantages:
larger spatial steric hindrance and torque, higher hole mobility, better solubility, higher efficiency and longer lifetime, and higher lateral resistance. Therefore, when paired with the P-dopant material currently used in the market, the lateral leakage of the device is significantly reduced and the technical problem of client-side crosstalk is solved.

### Brief Description of Drawings

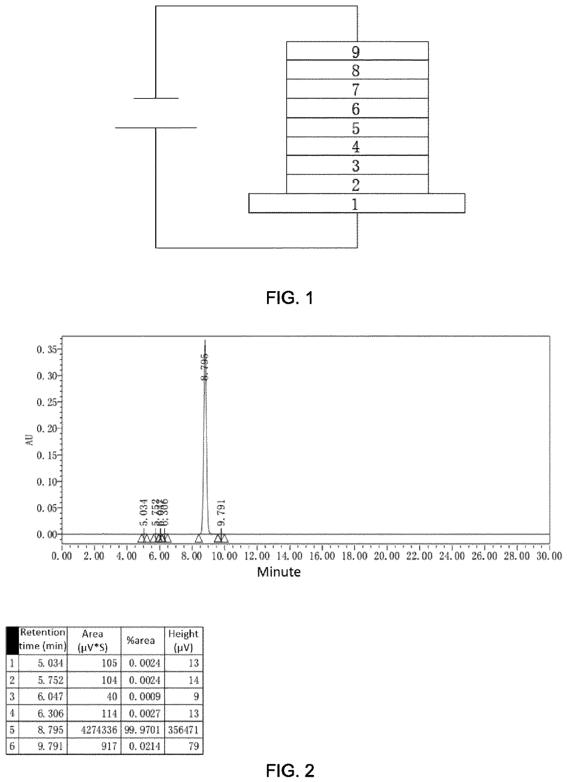

FIG. 1 is a structural schematic view of an organic electroluminescent device provided by the present disclosure;
   Numeral references: 1-anode, 2-hole injection layer, 3-first hole transport layer, 4-second hole transport layer, 5-emissive layer, 6-hole blocking layer, 7-electron transport layer, 8-electron injection layer, 9-cathode.
FIG. 2 is an HPLC of compound 10 prepared in Example 1 of the present disclosure.

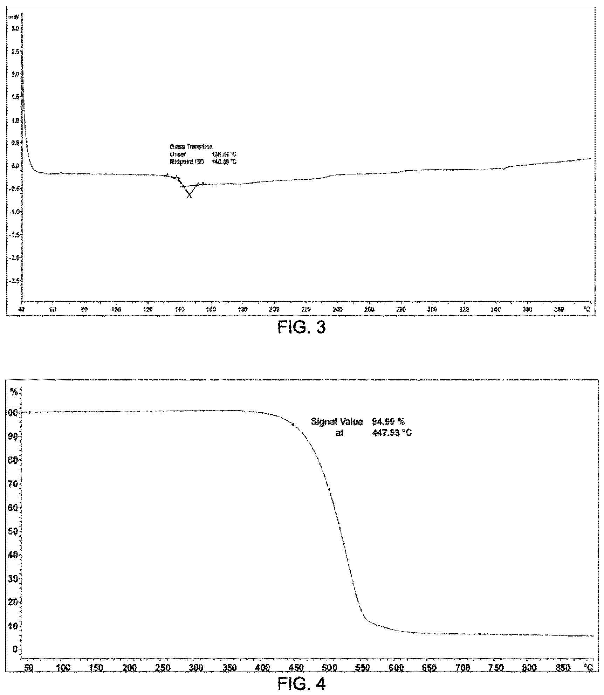

FIG. 3 shows the DSC profile of compound 10 prepared in Example 1 of the present disclosure, as can be seen from FIG. 3, the Tg value of compound 10 is 140.59°C.
FIG. 4 is the TGA profile of compound 10 prepared in Example 1 of the present disclosure, as can be seen from FIG. 4, the heat loss temperature Td value is 447.93°C.

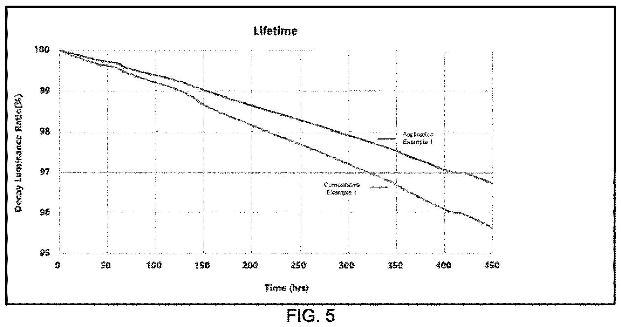

FIG. 5 is the lifetime view of the organic electroluminescent devices in Application Example 1 and Comparative Example 1 of the present disclosure; as can be seen from FIG. 5, the organic electroluminescent devices prepared in Application Example 1 and Comparative Example 1 of the present disclosure have T97% lifetimes of 407h and 319h, respectively.

### Detailed Description of Embodiments

Various embodiments are further illustrated and described below. It should be understood that the description herein is not intended to limit the claims to the particular aspects described. Rather, it is intended to cover alternatives, modifications, and equivalents as can be included within the spirit and scope of the present disclosure as defined by the appended claims.

As used herein, a "Ca to Cb" hydrocarbon group is defined as hydrocarbon groups with a carbon number of "a" (inclusive) to "b" (inclusive). As used herein, "a and/or b" denotes "a" or "b" or "a and b".

As used herein, in "substituted" or "unsubstituted", the term "substituted" means that at least one hydrogen in the group is recoordinated with a deuterium, hydrocarbon, hydrocarbon-derivative, halogen, or cyano (-CN) group. The term "unsubstituted" means that at least one hydrogen in the group is not recoordinated with deuterium, hydrocarbon, hydrocarbon-derivative, halogen or cyano (-CN). Examples of hydrocarbon or hydrocarbon-derivative groups can include, but are not limited to, C1 to C30 alkyl, C2 to C30 alkenyl, C2 to C30 alkynyl, C6 to C30 aryl, C5 to C30 heteroaryl, C1 to C30 alkylamino, C6 to C30 arylamino, C6 to C30 heteroarylamino, C6 to C30 arylheteroarylamino, and the like.

C1-C4 alkyl in the present disclosure indicates methyl, ethyl, n-propyl, isopropyl, n-butyl, sec-butyl, isobutyl, tert-butyl; and C1-C4 deuterated alkyl indicates a group obtained by substituting any number of hydrogens in methyl, ethyl, n-propyl, isopropyl, n-butyl, sec-butyl, isobutyl, tert-butyl with deuterium.

Where specific conditions are not indicated in the Examples, conventional conditions or conditions recommended by the manufacturer are followed. The reagents or instruments used without indication of the manufacturer are conventional products that can be purchased commercially.

### Example 1

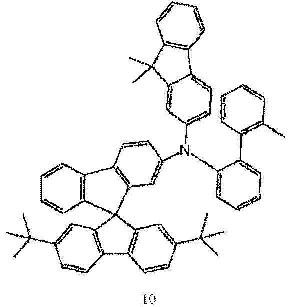

Compound 10 was synthesized as follows: 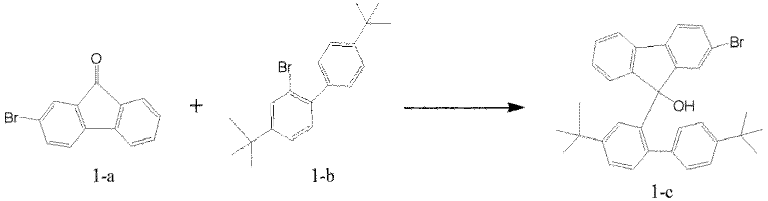 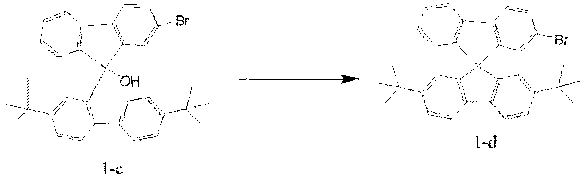 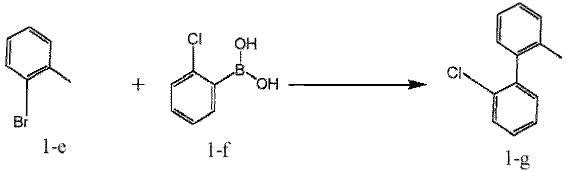 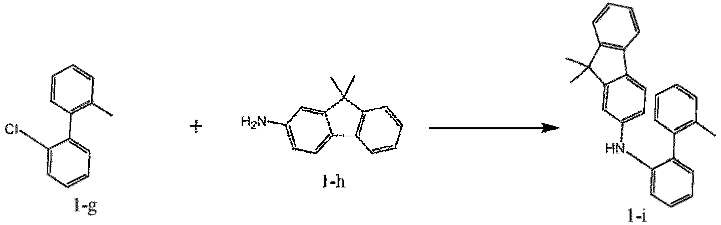 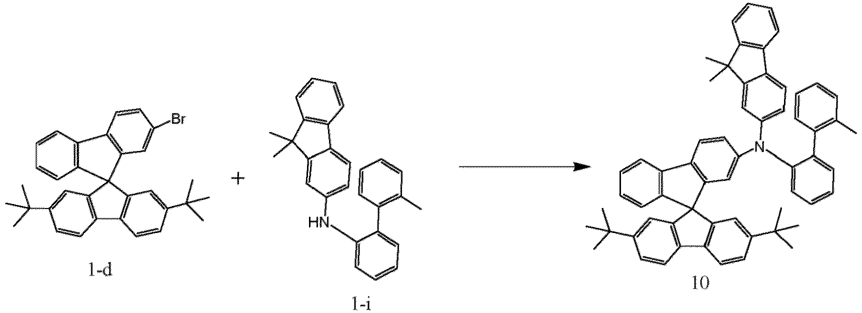
(1) adding compound 1-b (1eq, 344.11g/mol, 14.5mmol, 5g), anhydro THF (50g, 10 times the mass of compound 1-b) to the reaction flask under nitrogen protection, using liquid nitrogen to cool down to -78°C, adding n-butyl lithium (1.1eq, 15.95mmol) dropwise, after reacting for 30min, mixing compound 1-a (4.74g, 257.97g/mol, 14.5mmol), anhydrous THF (47.4g, 10 times the mass of compound 1-a) evenly and adding dropwise to the resultant, and continuing the reaction at -78 °C for 2h; then adding ammonium chloride solution to quench, adding dichloromethane and water after slowly returning to the room temperature for extraction and liquid separation, washing with water for several times after dichloromethane phase was separated, drying by anhydrous sodium sulfate, concentrating under reduced pressure to obtain the crude product of compound 1-c, and purifing through the column chromatography to obtain the pure product of compound 1-c (8.45g, yield is 55.6%), wherein MS (El): 524 (M⁺);
(2) adding compound 1-c (8g, 15.2mmol) to isopropanol (80g, 10 times the mass of compound 1-c), then adding hydrochloric acid (1M, 80 g, 10 times the mass of compound 1-c) dropwise, and carrying out heating azeotropic reaction for 5h, then distilling the resultant under reduced pressure to remove isopropanol, and carrying out suction filtration to obtain compound 1-d (7.39 g, yield is 96.1%), wherein MS (El): 506 (M⁺);
(3) adding compound 1-e (37.97g, 0.222mol, 1eq), compound 1-f (34.71g, 0.222mol, 1eq), potassium carbonate (61.36g, 0.444mol, 2eq), and toluene/ethanol/water (500ml+250ml+150ml) to a 1L three-necked flask, under N2 protection, adding tetrakis(triphenylphosphine)palladium (5.13g, 4.44mmol, 0.02eq), heating after adding and performing reflux reaction, and using HPLC to monitor compound 1-e ≤1%; stopping the reaction, adding 150ml water and stirring for liquid separation, extracting the aqueous phase with DCM (100 ml * 2), combining the organic phase, filtering through silica gel powder to perform suction filtration, concentrating the filtrate to dryness under reduced pressure, adding 200ml PE, stirring and performing crystallization for 1-2h, performing suction filtration, and performing blowing dry on a cake at 65°C to obtain compound 1-g (33.35g, light yellow solid, yield 74.3%);
(4) adding compound 1-g (33g, 0.16mol, 1eq), compound 1-h (33.49g, 0.16mol, 1eq), sodium tert-butoxide (30.75g, 0.32mol, 2eq), XPhos (3.05g, 6.4mmol, 0.04eq) and toluene (400ml) to a 1L three-necked flask, under N2 protection, adding palladium acetate (0.72g, 3.2mmol, 0.02eq), after finishing adding, heating and performing reflux reaction, and using HPLC to monitor compound 1-g ≤1%; stopping the reaction, adding 400ml water, stirring and performing liquid separation, extracting the aqueous phase with DCM (200ml*2), combining the organic phases, filtering through silica gel to perform suction filtration, concentrating the filtrate to dryness under reduced pressure, adding 200ml PE, cooling and stirring and performing crystallization for 2h, performing suction filtration, and performing blowing dry on a cake at 85°C to obtain gray solid 1-i (34.96g, yield 60.3%); and
(5) under nitrogen protection, adding compound 1-d (7g, 13.8mmol), compound 1-i (1.1eq, 375.51g/mol, 15.2 mmol, 5.7g), sodium tert-butoxide (1.1eq, 96.1g/mol, 15.2mmol, 1.46g), Pd2(dba)3 (5%eq, 915.72g/mol, 0.69mmol, 0.63g), tri-tert-butyl phosphine (5%eq, 202.317g/mol, 0.69mmol, 0.14g), and toluene (70g, 10 times the mass of compound 1-d) to the reaction flask, after finishing adding, heating and performing reflux reaction for 5h; after the reaction being completed, cooling to room temperature, then adding water and stirring for 15 min, and filtering to obtain the filtrate; separating the filtrate to obtain the organic phase, drying the organic phase by anhydrous magnesium sulfate and spun-drying, and prurifying by column chromatography to obtain the high-purity compound 10 (7.0g, yield 63.5%), wherein through ESI-MS (m/z) (M+), the theoretical value was 802.12, and the measured value was 801.78; and elemental analysis results (molecular formula C61H55N) were as follows, theoretical value: C was 91.34, H was 6.91, and N was 1.75; and the measured value: C was 91.32, H was 6.93, N was 1.75.

The following product compounds were obtained in a similar manner.

**Table 1**

| Example | Raw material 1 | Raw material 2 | Product | recovery rate |
|---|---|---|---|---|
| 2 | 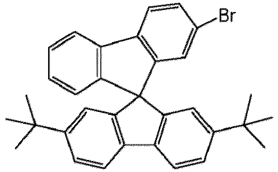 | 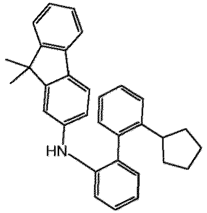 | 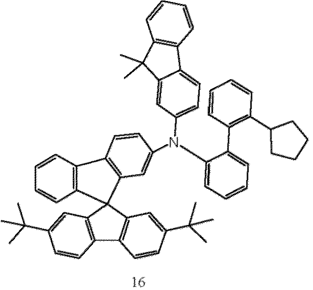 | 62.7% |
| 3 | 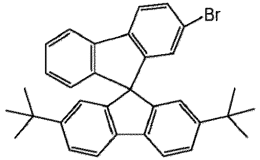 | 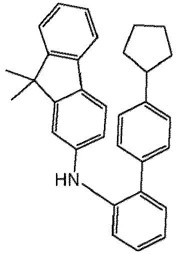 | 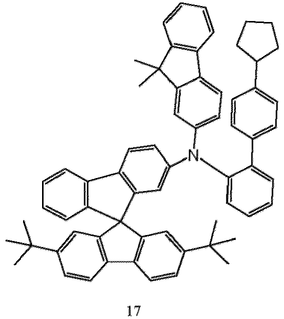 | 64.2% |
| 4 | 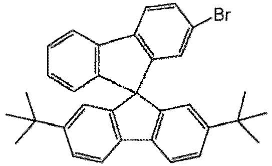 | 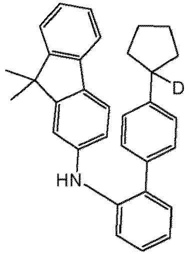 | 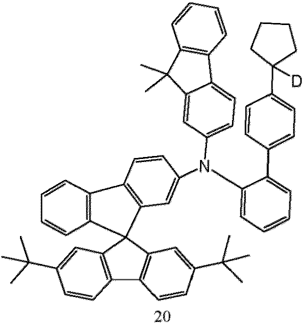 | 59.3% |
| 5 | 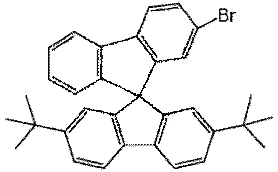 | 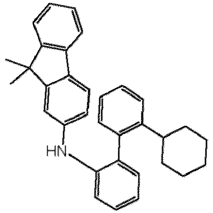 | 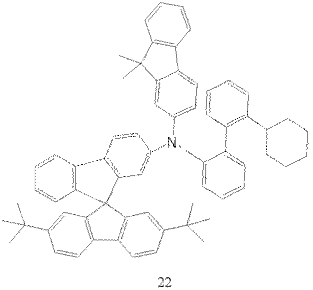 | 66.7% |
| 6 | 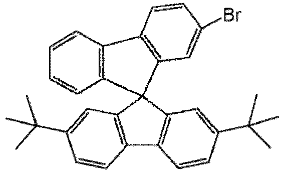 | 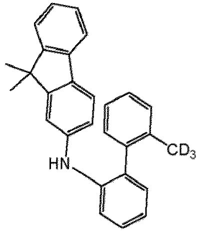 | 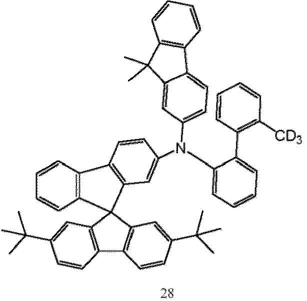 | 68.5% |
| 7 | 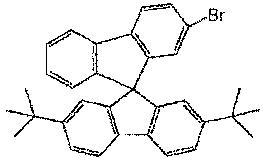 | 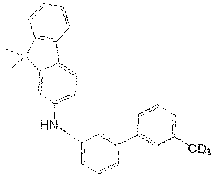 | 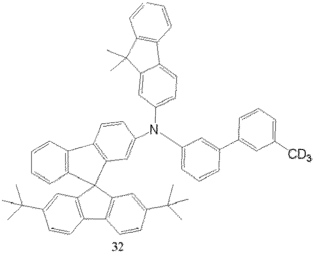 | 71.2% |
| 8 | 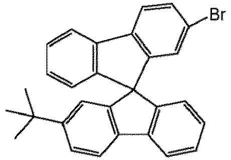 | 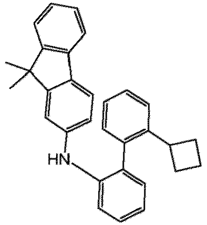 | 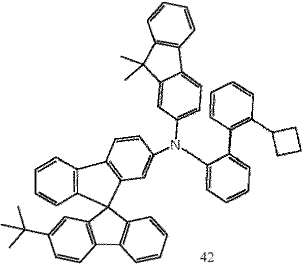 | 65.5% |
| 9 | 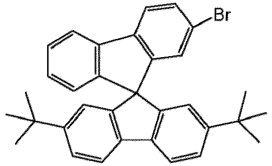 | 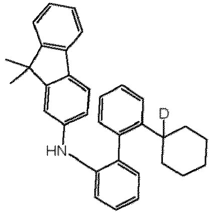 | 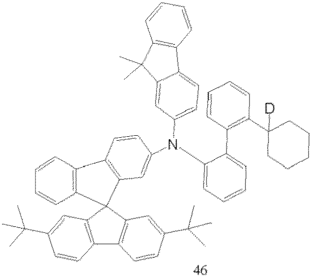 | 68.3% |
| 10 | 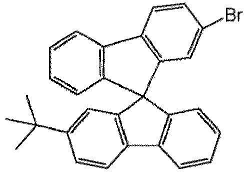 | 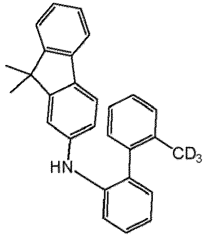 | 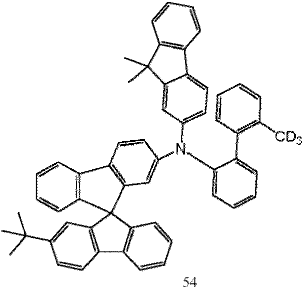 | 67.6% |
| 11 | 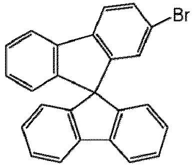 | 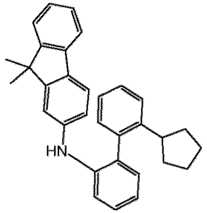 | 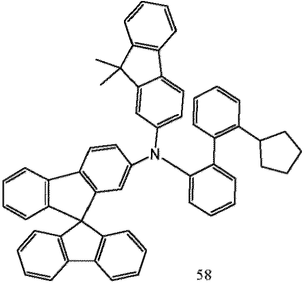 | 58.9% |
| 12 | 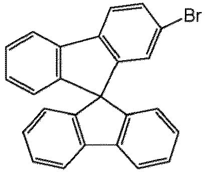 | 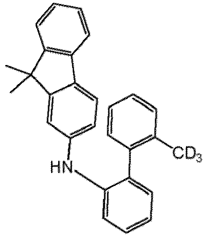 | 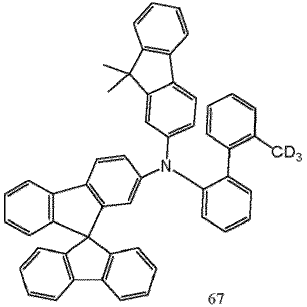 | 63.2% |
| 13 | 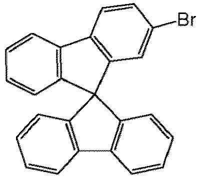 | 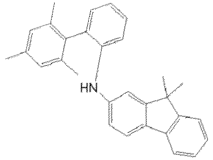 | 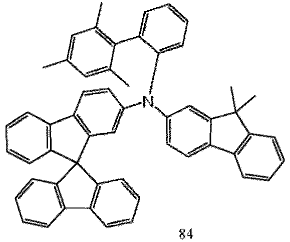 | 67.8% |
| 14 | 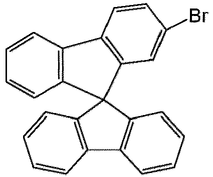 | 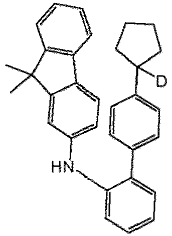 | 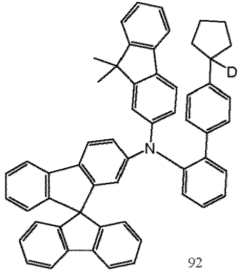 | 65.1% |

The synthetic identification of the compounds prepared above is shown in Table 2 below.

**Table 2**

| Compound | FD Quality | Compound | FD Quality |
|---|---|---|---|
| 10 | m/z=801.78 (C61H55N=802.12) | 42 | m/z=785.59 (C60H51N=786.07) |
| 16 | m/z=855.92 (C65H61N=856.21) | 46 | m/z=871.45 (C66H62DN=871.24) |
| 17 | m/z=855.73 (C65H61N=856.21) | 54 | m/z=749.22 (C57H44D3N=749.03) |
| 20 | m/z=857.30 (C65H60DN=857.22) | 58 | m/z=743.78 (C57H45N=743.99) |
| 22 | m/z=869.81 (C66H63N=870.24) | 67 | m/z=693.36 (C53H36D3N=692.92) |
| 28 | m/z=805.26 (C61H52D3N=805.14) | 84 | m/z=717.55 (C55H43N=717.96) |
| 32 | m/z=805.47 (C61H52D3N=805.14) | 92 | m/z=745.31 (C57H44DN=745.00) |

### Material property test:

The compounds 10, 16, 17, 20, 22, 28, 32, 42, 46, 54, 58, 67, 84, 92 of the present disclosure were tested for their heat loss temperature Td and glass transition temperature Tg, and the results of the tests are shown in Table 3 below.

Note: The thermal weight loss temperature Td is the temperature at which 5% of the weight is lost in a nitrogen atmosphere, and was determined on a TGAN-1000 thermogravimetric analyzer with a nitrogen flow rate of 10mUmin, and the glass transition temperature Tg was determined by differential scanning calorimetry (DSC, Shinko DSC N-650) with a heating rate of 10°C/min.

**Table 3:**

| Test materials (compounds) | Td (°C) | Tg (°C) |
|---|---|---|
| 10 | 447.93 | 140.59 |
| 16 | 431.25 | 124.33 |
| 17 | 452.62 | 133.51 |
| 20 | 458.77 | 146.28 |
| 22 | 465.78 | 161.56 |
| 28 | 455.57 | 142.56 |
| 32 | 460.98 | 151.08 |
| 42 | 438.79 | 156.27 |
| 46 | 441.55 | 160.59 |
| 54 | 449.62 | 128.33 |
| 58 | 453.37 | 134.67 |
| 67 | 461.22 | 141.26 |
| 84 | 463.39 | 148.54 |
| 92 | 455.57 | 157.38 |

From the above data, it can be seen that the compounds of the present disclosure have high Td and Tg values, indicating that they have excellent thermal stability, and their application in organic electroluminescence devices can effectively extend the service life of organic electroluminescence devices, thereby obtaining better utilization effects.

### Device performance test:

### Application Example 1

ITO was used as the anode substrate material for the reflective layer, and the surface thereof was treated with water, acetone, and N₂ plasma in sequence;
10nm of the compound prepared in Example 1 of the present disclosure doped with 3% wtHAT-CN was deposited on the ITO anode substrate to form hole injection layer (HIL);
evaporation of 100 nm of the compound 10 prepared in Example 1 of the present disclosure was performed on the hole injection layer (HIL) to form a hole transport layer (HTL);
vacuum evaporation of EB-1 was performed on the hole transport layer (HTL) to form an electron blocking layer (EBL) with a thickness of 10 nm;
co-evaporation of GH-1 and GH-2 as the main luminescent materials was performed in a 5:5 mass ratio, and GD-1 as a dopant material (the amount of GD-1 was 8% of the total weight of GH-1 and GH-2) was evaporated to form a emissive layer with a thickness of 20nm on the electron barrier layer (EBL);
evaporation of HB-1 was performed on the emissive layer to obtain a hole blocking layer (HBL) with a thickness of 20nm;
co-evaporation of ET-1 and LiQ was performed on the hole blocking layer (HBL) in a mass ratio of 5:5 to obtain an electron transport layer (ETL) with a thickness of 30 nm;
evaporation of the mixture of magnesium (Mg) and silver (Ag) in a 9:1 mass ratio was performed on the electron transport layer (ETL) to form an electron injection layer (EIL) with a thickness of 50 nm; and then
evaporation of silver (Ag) was performed on the electron injection layer to form a cathode with a thickness of 100nm, and DNTPD with a thickness of 50 nm was deposited on the sealing layer of the above-mentioned cathode; in addition, the cathode surface was sealed with a UV-hardening adhesive and a seal cap containing a dehumidifying agent, so as to protect the organic electroluminescent device from being affected by oxygen or moisture in the atmosphere. Then, the organic electroluminescent device is obtained.

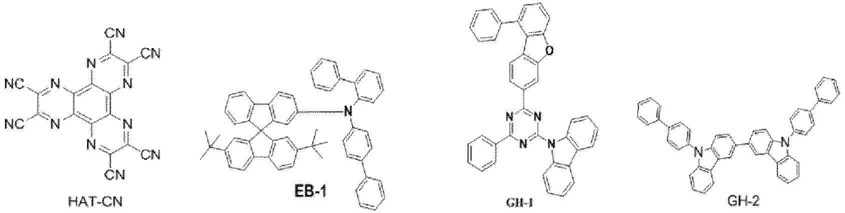

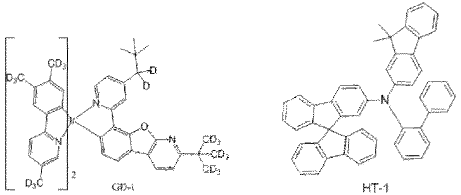

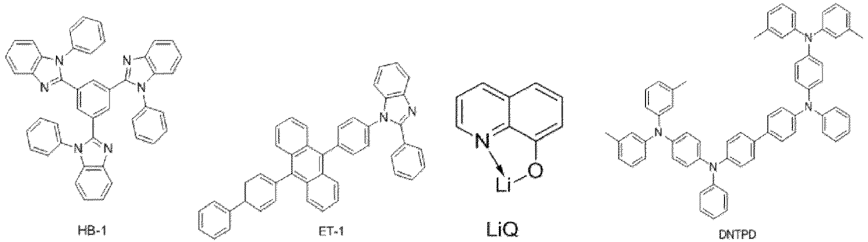

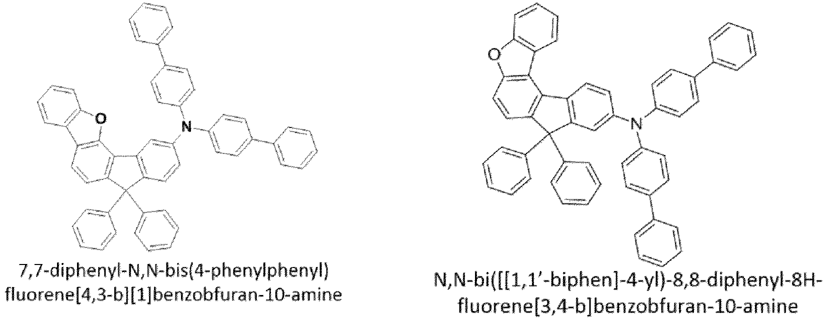

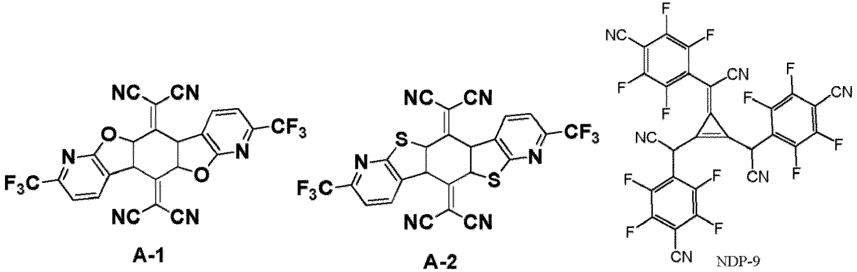

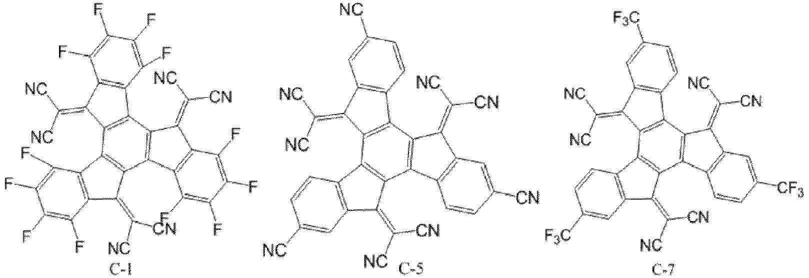

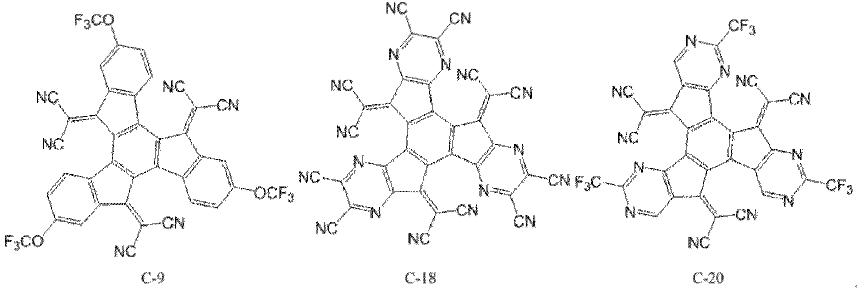

### Application Example 2-14

Organic electroluminescent compounds 16, 17, 20, 22, 28, 32, 42, 46, 54, 58, 67, 84, 92 in Examples 2-14 of the present disclosure were used to replace compound 10 in the hole transport region of Application Example 1 respectively, and the other portions were consistent with Application Example 1, whereby organic electroluminescent devices of Application Examples 2-14 were produced.

### Application examples 15-16

Compounds A-1 and A-3 were used to replace HAT-CN in Application Example 1 to form a hole injection layer (HIL) respectively, and the rest were the same as in Application Example 1.

Compound A-1 was synthesized as follows: 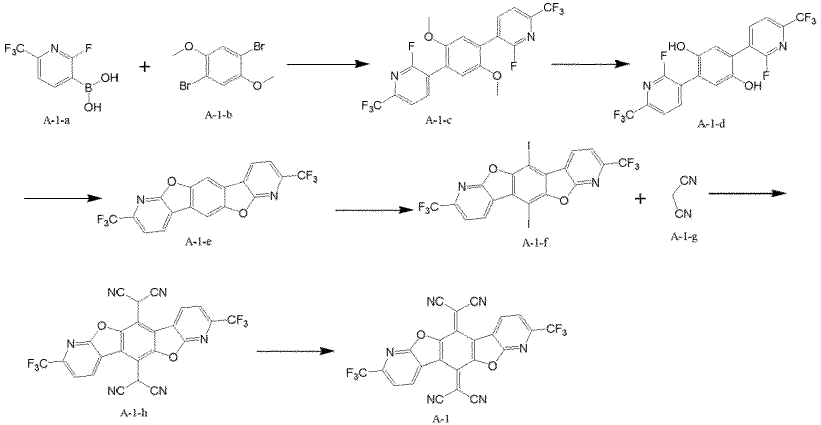
under the nitrogen protection, adding boronic acid compound A-1-a (41.78g, 0.2mol, 2eq), A-1-b (29.596g, 0.1mol, 1eq) to the reaction flask, then adding toluene (600ml), ethanol (150ml), potassium carbonate (41.4g, 0.3mol, 3eq), water (150ml), and tetrakis(triphenylphosphine)palladium (2.31g, 0.002mol, 2%eq), after the addition was completed, heating the reaction solution to carry out reflux reaction for 6h, turning off the heating, cooling to room temperature and adding 200ml of water, separating the resultant, drying the organic phase by silica gel, concentrating to dryness, and performing column chromatography to obtain productA-1-c (about 39.47g, yield was 85%);
under nitrogen protection, adding intermediate A-1-c (39.47 g, 0.085 mol, 1eq) to the reaction flask, adding 400 ml dichloromethane, cooling down to 0°C, adding boron tribromide (63.9g, 0.255mol, 3eq) dropwise, after addition was completed, stirring and reacting overnight, adding water dropwise to the reaction, separating the resultant, and after washing the organic phase with water, separating the resultant, and concertrating to dryness, and purifying through the column chromatography to obtain product A-1-d (about 29.67g, yield was 80%);
adding intermediate A-1-d (29.67g, 0.068mol, 1eq) to the reaction flask, then adding NMP (350ml), potassium carbonate (28.152g, 0.204mol, 3eq), after the addition was completed, heating the reaction solution to 190°C and reacting for 4h, in which the solid was precipitated after adding water, performing suction filtration, washing the cake with water, and PE and drying to obtain product A-1-e (about 22.9g, yield was 85%);
adding intermediate A-1-e (22.9g, 0.0578mol, 1eq) to acetic acid (240 mL), then adding iodine (29.34g, 0.1156mol/2eq), iodic acid (20.34 g, 0.1156mol, 2eq), carbon tetrachloride (15mL), concentrated sulfuric acid (2mL), and distilled water (20mL), heating to 65°C and stirring for 48h, cooling to room temperature, filtering, and washing the solid with acetic acid, ethyl acetate and petroleum ether to obtain productA-1-f (about 24.35g, yield was 65%); and
under nitrogen protection, adding malononitrile (6.2g, 0.09393mol, 2.5eq) into THF (300mL), adding sodium hydride (4.5g, 0.18785mol, 5eq) at 0°C, stirring for 10min and then heating to room temperature, then adding intermediate A-1-f (24.35g, 0.03757mol, 1eq) and tetrakis(triphenylphosphine)palladium (2.17g, 0.00188mol, 5%eq), raising to 75°C and reacting for 6h; cooling to room temperature, adding 1N dilute hydrochloric acid (200mL), with a large amount of solid precipitated, filtering and washing the cake with water and petroleum ether, and then drying to obtain productA-1-h (about 15.73g, yield was 80%); and
under nitrogen protection, adding intermediate A-1-h (15.73g, 0.03mol, 1eq) into DCM (200mL), then adding PIFA (14.19g, 0.033mol, 1.1eq), stirring at room temperature overnight, and purifying the reaction solution directly by preparative sand column chromatography to obtain compound A-1 (about 14.1g, yield was 90%).

Compounds A-3 were synthesized as follows: 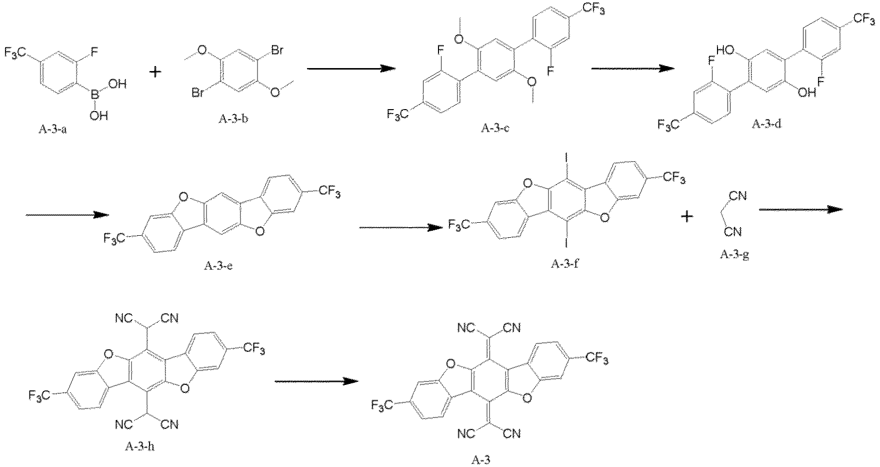
under nitrogen protection, adding boronic acid compound A-3-a (41.58g, 0.2mol, 2eq), and halogenated compound A-3-b (29.596g, 0.1mol, 1eq) to the reaction flask, then adding toluene (600ml), ethanol (150ml), potassium carbonate (41.4g, 0.3mol, 3eq), water (150ml), and tetrakis(triphenylphosphine)palladium (2.31g, 0.002mol, 2%eq), after the addition was completed, heating the reaction solution to carry out reflux reaction for 6h, turning off the heating, cooling to room temperature and adding 200ml of water, separating the resultant, drying the organic phase by silica gel, concentrating to dryness, and purifying by column chromatography to obtain productA-3-c (about 39.3g, yield was 85%);
under nitrogen protection, adding intermediate A-3-c (39.3g, 0.085mol, 1eq) into the reaction flask, adding 400ml of dichloromethane, cooling to 0°C, adding boron tribromide (63.9g, 0.255mol, 3eq) dropwise, then stirring and reacting overnight, adding water dropwise to the reaction, separating the resultant, and after washing the organic phase with water, separating the resultant, concentrating to dryness, and purifying by column chromatography to obtain product A-3-d (about 29.53g, yield was 80%);
adding intermediate A-3-d (29.53g, 0.068mol, 1eq) into the reaction flask, adding NMP (350ml), potassium carbonate (28.152g, 0.204mol, 3eq), after the addition was completed, the heating the reaction solution to 190°C and reacting for 4h, with a solid precipitated after the addition of water, performing suction filtration, and washing the cake with water, and PE, and drying to obtain product A-3-e (about 22.79g, yield was 85%);
adding intermediate A-3-e (22.79g, 0.0578mol, 1eq) to acetic acid (240mL), then adding iodine (29.34g, 0.1156mol, 2eq), iodic acid (20.34g, 0.1156mol, 2eq), carbon tetrachloride (15mL), concentrated sulfuric acid (2mL), and distilled water (20mL), heating to 65°C and stirring for 48h, cooling to room temperature, filtering, and washing the solid with acetic acid, ethyl acetate and petroleum ether to obtain productA-3-f (about 24.27g, the yield was 65%);
under nitrogen protection, adding malononitrileA-3-g (6.2g, 0.09393mol, 2.5eq) into THF (300 mL), adding sodium hydride (4.5 g, 0.18785 mol, 5eq) at 0°C, stirring for 10min and then raising to room temperature, adding the intermediateA-3-f (24.27g, 0.03757mol, 1eq) and the tetrakis(triphenylphosphine)palladium (2.17g, 0.00188mol, 5%eq), heating to 75°C and reacting for 6h, cooling to room temperature, adding 1N dilute hydrochloric acid (200mL), with a large amount of solid precipitated, filtering and washing with water and petroleum ether to wash the cake, and drying to obtain productA-3-h (about 15.67g, the yield was 80%); and
under nitrogen protection, adding intermediate A-3-h (15.67g, 0.03mol, 1eq) into DCM (200mL), then adding PIFA (14.19g, 0.033mol, 1.1eq) and stirring overnight at room temperature, and purifying the reaction solution directly by preparative sand column chromatography to obtain compound A-3 (about 14.05g, yield 90%).

### Application Example 17

Compound NDP-9 was used instead of HAT-CN in Application Example 1 to form a hole injection layer (HIL), and the rest was the same as in Application Example 1.

### Application Example 18-23

Compounds C-1, C-5, C-7, C-9, C-18, and C-20 were used to replace HAT-CN in Application Example 1 to form a hole injection layer (HIL) respectively, and the rest were the same as in Application Example 1.

### Comparative Examples 1-3

The difference between Comparative Examples 1-3 and Application Example 1 was that HT-1, 7,7-diphenyl-N,N-bis(4-phenylphenyl)fluorene[4,3-b] [1]benzofuran-10-amine, and N,N-bis([1,1'-biphenyl]-4-yl)-8,8-diphenyl-8H-fluorene[3,4-b]benzofuran-10-amine were used to replace organic electroluminescent compounds 10 in the hole transport region in Application Example 1 respectively, and the rest are the same as in Application Example 1.

The organic electroluminescent devices prepared by Application Examples 1-14 and Comparative Examples 1-3 were tested separately, and the test results are shown in Table 4.

The organic electroluminescent devices prepared by Application Examples 15-16 were tested separately, and the test results are shown in Table 5.

The organic electroluminescent device prepared by Application Examples 17 was tested separately, and the test results are shown in Table 6.

The organic electroluminescent devices prepared by Application Examples 18-23 were tested separately, and the test results are shown in Table 7.

Note: T97% is the time when the luminous brightness of the device is reduced to 97% of the initial brightness, and the test equipment is TEO luminous device lifespan test system.

**Table 4**

| Experiment group | Hole transport layer material | Voltage (V) | Luminous efficiency (Cd/A) | T97%/h | Lighting Color |
|---|---|---|---|---|---|
| Comparative Example1 | HT-1 | 4.4 | 8.3 | 319 | blue |
| Comparative Example2 | 7,7-diphenyl-N,N-bis(4-phenylphenyl)fluorene[4,3-b] [1]benzofuran-10-amine | 4.8 | 7.8 | 327 | blue |
| Comparative Examples3 | N,N-bis([[1,1'-biphenyl]-4-yl)-8,8-diphenyl-8H-fluoreno[3,4-b]benzofuran-10-amine | 4.6 | 8.1 | 315 | blue |
| Application Example 1 | 10 | 2.4 | 14.6 | 407 | blue |
| Application Example 2 | 16 | 2.1 | 14.3 | 411 | blue |
| Application Example 3 | 17 | 2.0 | 15.1 | 403 | blue |
| Application Example 4 | 20 | 2.2 | 15.0 | 413 | blue |
| Application Examples5 | 22 | 2.3 | 14.7 | 417 | blue |
| Application Example 6 | 28 | 2.1 | 15.2 | 408 | blue |
| Application Example 7 | 32 | 2.4 | 15.3 | 405 | blue |
| Application Example 8 | 42 | 2.3 | 15.1 | 410 | blue |
| Application Example 9 | 46 | 2.0 | 14.7 | 404 | blue |
| Application Example10 | 54 | 2.1 | 14.5 | 417 | blue |
| Application Example 11 | 58 | 2.0 | 14.8 | 414 | blue |
| Application Example 12 | 67 | 2.2 | 15.1 | 412 | blue |
| Application Example 13 | 84 | 2.1 | 15.3 | 409 | blue |
| Application Example 14 | 92 | 2.3 | 14.9 | 404 | blue |

As can be seen from Table 4 above, applying the compounds of the present disclosure to organic electroluminescent devices and using them as hole transport layer materials can lead to a certain magnitude of improvement in the luminous efficiency of the organic electroluminescent devices, as well as a decrease in the start-up voltage, such that the power consumption of the devices decreases, and the lifespan of the devices increases significantly.

**Table 5**

| Experiment Group | Hole injection material | Voltage (V) | Luminous efficiency (Cd/A) | T97%/h | Lighting Color |
|---|---|---|---|---|---|
| Application Example 1 | HAT-CN | 2.4 | 14.6 | 407 | blue |
| Application Example15 | Compound A-1 | 2.2 | 14.7 | 409 | blue |
| Application Example16 | Compound A-3 | 2.1 | 15.2 | 413 | blue |

As can be seen from Table 5 above, applying compounds A-1 and A-3 in organic electroluminescent devices in which the compounds of the present disclosure are used as hole transport layer materials to act as hole injection materials can further improve the luminous efficiency of organic electroluminescent devices, decrease the power consumption of the devices and increase the device lifespan, indicating that compounds A-1, A-3 and compound 10 of the present disclosure have a good cooperation role in the devices.

**Table 6**

| Experiment Group | Hole injection material | Voltage (V) | Luminious efficiency (Cd/A) | T97%/h | Lighting Color |
|---|---|---|---|---|---|
| Application Example 1 | HAT-CN | 2.4 | 14.6 | 407 | blue |
| Application Example17 | NDP-9 | 2.1 | 15.1 | 414 | blue |

As can be seen from Table 6 above, compound NDP-9 is applied to an organic electroluminescent device in which the compound of the present disclosure is used as a hole transport layer material to act as a hole injection material, such that the luminious efficiency of the organic electroluminescent device can be further improved, the power consumption of the device can be reduced, and the device lifespan can be improved, indicating that the compound NDP-9 and the compound 10 of the present disclosure have a good cooperating effect in the device.

**Table 7**

| Experiment Group | Hole injection material | Voltage (V) | Luminous efficiency (Cd/A) | T97%/h | Lighting color |
|---|---|---|---|---|---|
| Application Example 1 | HAT-CN | 2.4 | 14.6 | 407 | blue |
| Application Example18 | C-1 | 2.2 | 15.2 | 412 | blue |
| Application Example19 | C-5 | 2.1 | 14.7 | 409 | blue |
| Application Example20 | C-7 | 2.0 | 15.3 | 411 | blue |
| Application Example21 | C-9 | 2.3 | 15.0 | 408 | blue |
| Application Example22 | C-18 | 2.0 | 14.8 | 413 | blue |
| Application Example23 | C-20 | 2.2 | 14.7 | 411 | blue |

As can be seen from Table 7 above, compounds C-1, C-5, C-7, C-9, C-18, C-20 are applied to organic electroluminescent devices in which the compounds of the present disclosure are used as materials for the hole transport layer, and by using them as hole injection materials, the luminious efficiency of the organic electroluminescent devices can be further improved, the power consumption of the devices can be reduced, and the device lifespan can be improved, indicating that compounds C-1, C-5, C-7, C-9, C-18, C-20 and the compounds 10 of the present disclosure have a good cooperating effect in the devices.

## Claims

1. An organic electroluminescent device, comprising a cathode (9), an anode (1), and an organic layer formed between the cathode (9) and the anode (1), the organic layer contains an emissive layer (5);
a hole transport region is provided between the anode (1) and the emissive layer, wherein the hole transport region comprises a hole transport layer, or further comprises at least one of a hole injection layer (2) and an electron blocking layer;
an electron transport region selected from at least one of an electron transport layer (7), a hole blocking layer (6), and an electron injection layer (8) is provided between the emissive layer (5) and the cathode (9); wherein
at least one layer of the hole transport region contains an organic electroluminescent compound as shown in formula 1 below, 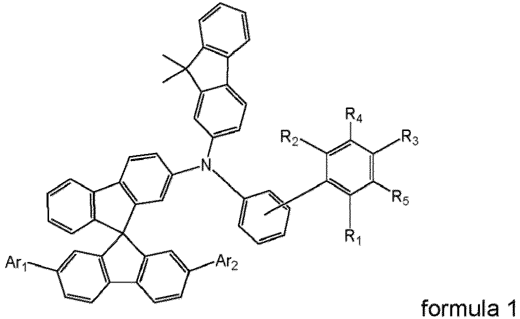
wherein
R₁-R₅ are each independently hydrogen, deuterium, deuterated or undeuterated alkyl, or deuterated or undeuterated cycloalkyl, and R₁-R₅ are not hydrogen at the same time; and
Ar, and Ar₂ are each independently hydrogen or an alkyl of C1-C4.

2. The organic electroluminescent device according to claim 1, wherein R₁-R₅ are each independently hydrogen, deuterated or undeuterated alkyl of C1-C4, deuterated or undeuterated cycloalkyl of C3-C6, and R₁-R₅ are not hydrogen at the same time, or R1-R3 are not hydrogen at the same time; and Ar₁, Ar₂ are each independently hydrogen or tert-butyl.

3. The organic electroluminescent device according to claim 1, wherein R₁-R₅ are each independently hydrogen, deuterated or undeuterated methyl, deuterated or undeuterated cyclobutyl, deuterated or undeuterated cyclopentyl, or deuterated or undeuterated cyclohexyl.

4. The organic electroluminescent device according to claim 1, wherein at least one layer in the hole transport region contains one or more of compounds shown below:

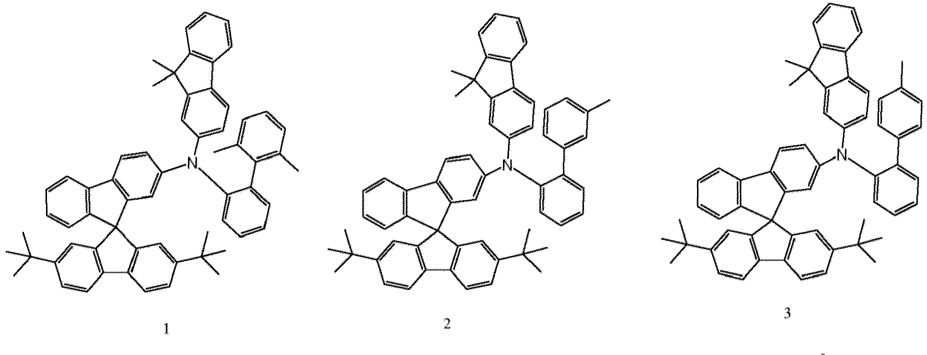

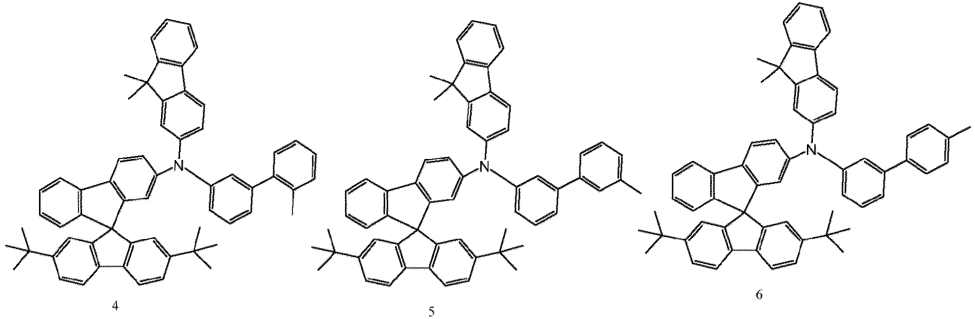

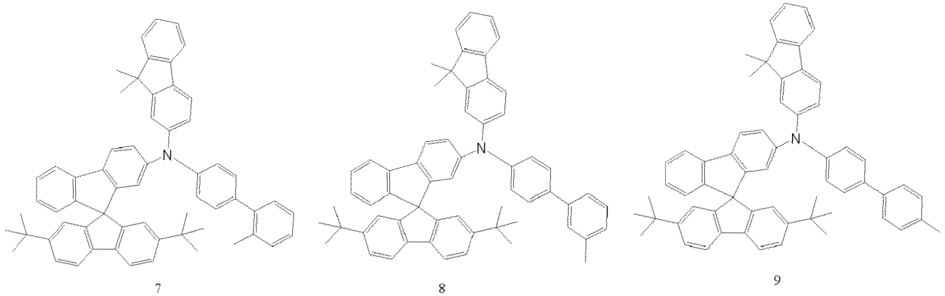

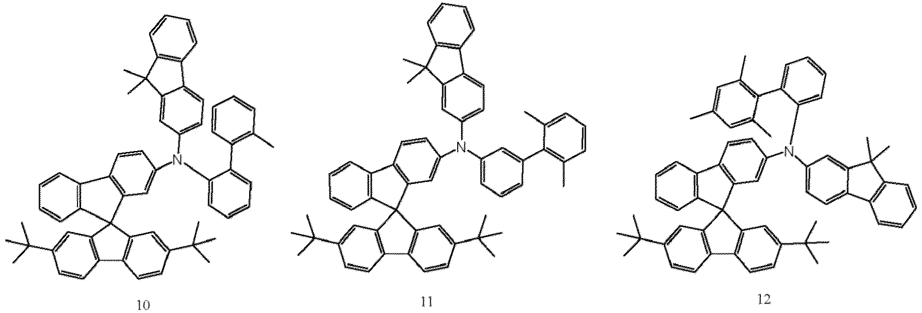

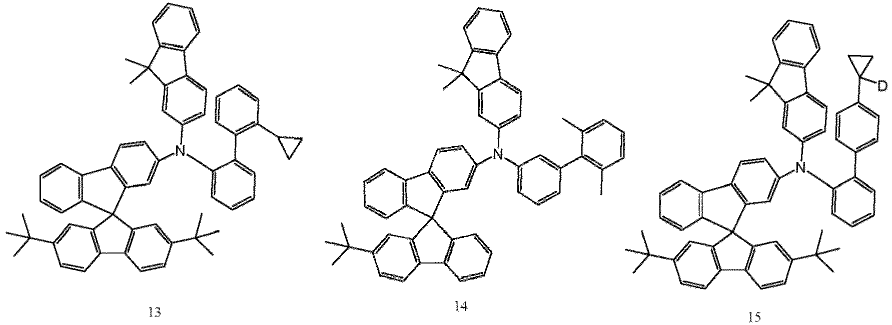

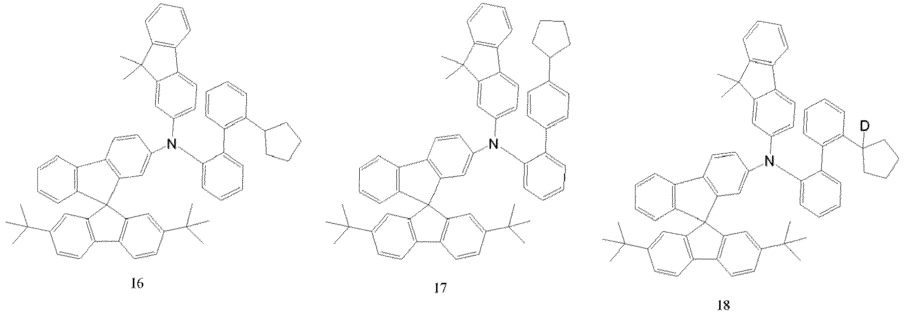

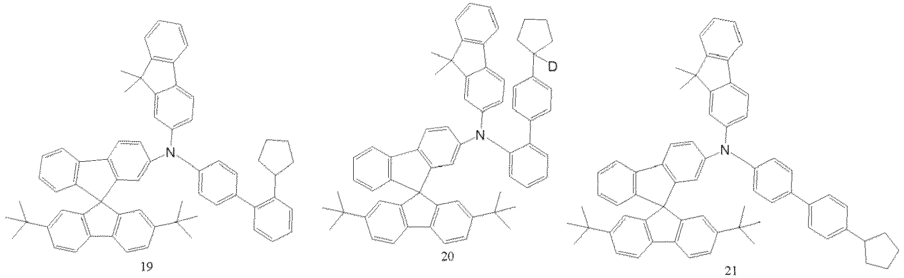

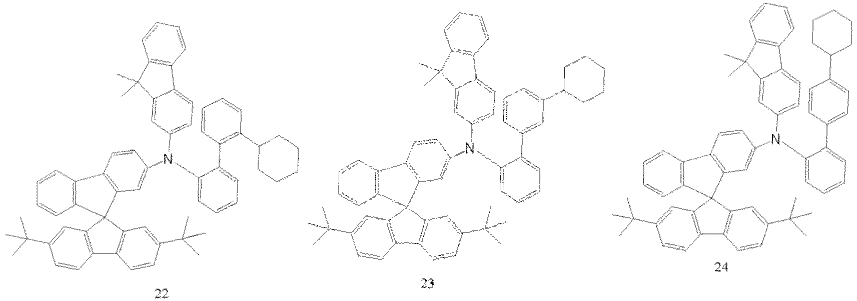

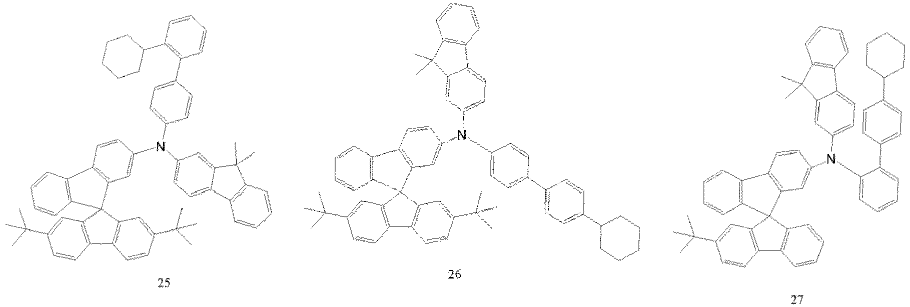

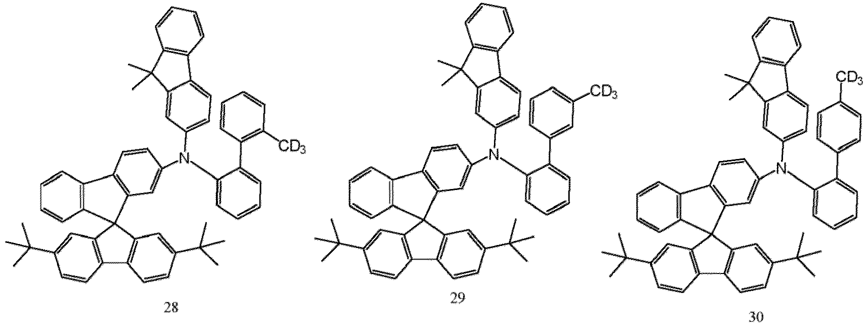

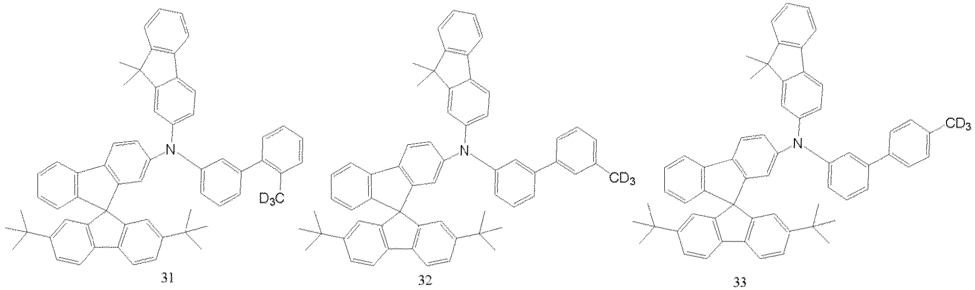

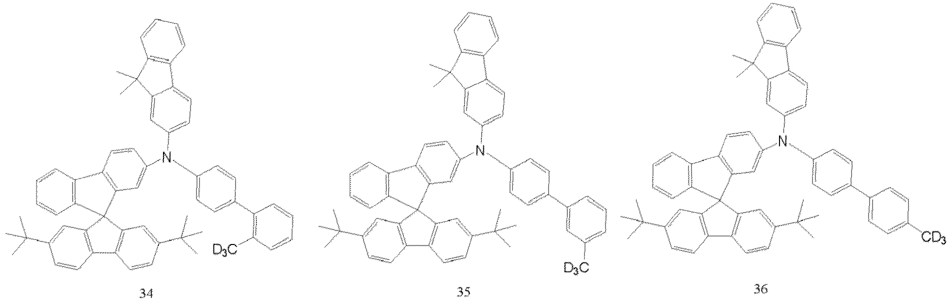

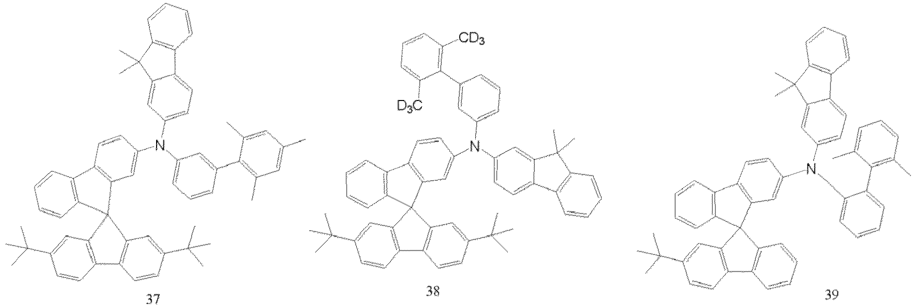

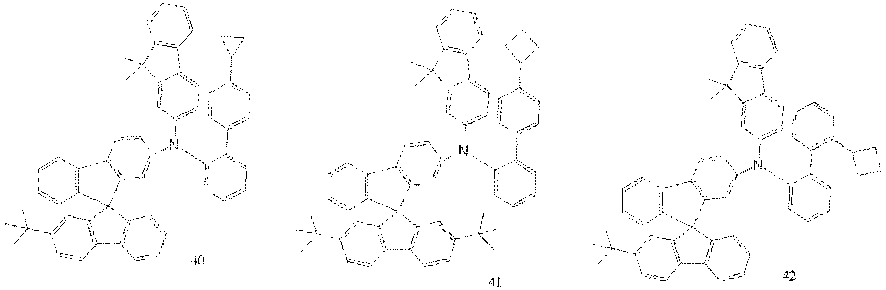

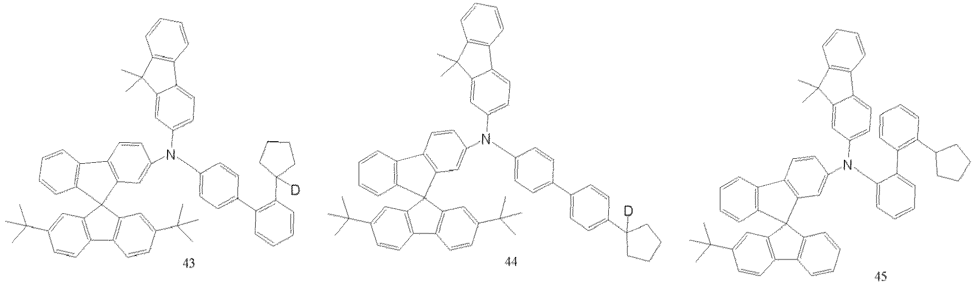

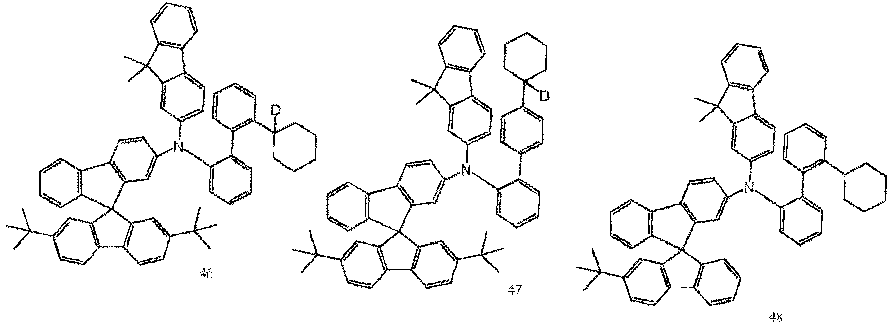

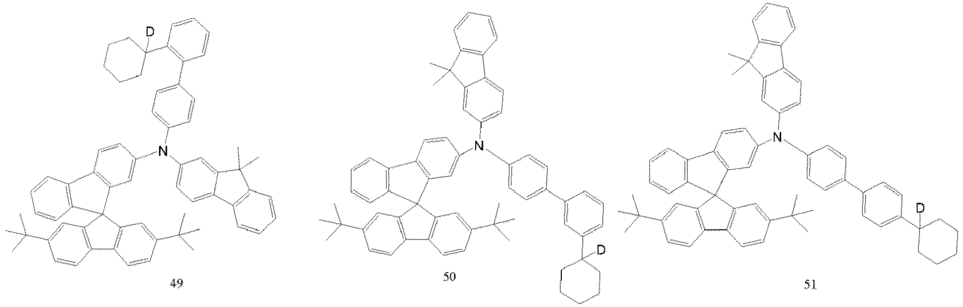

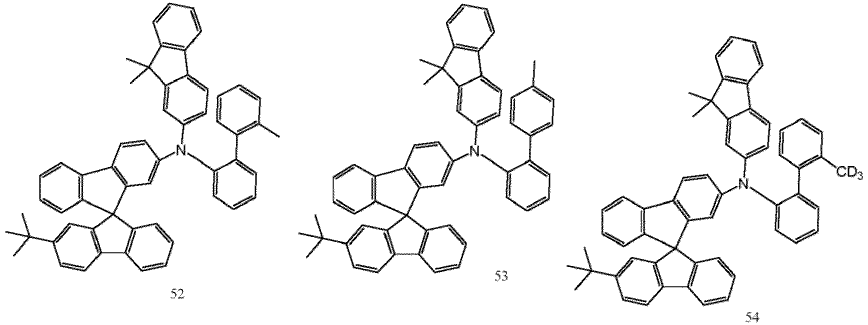

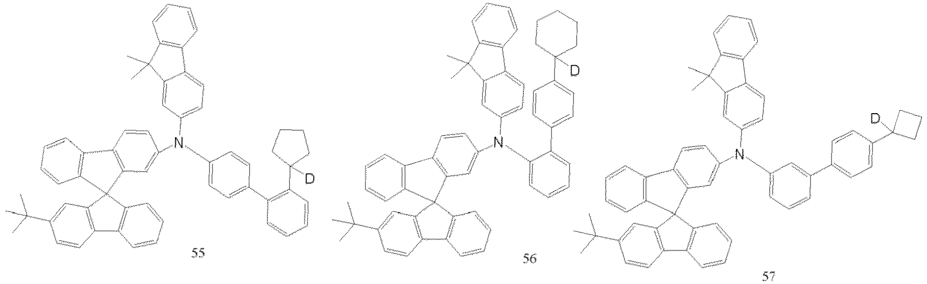

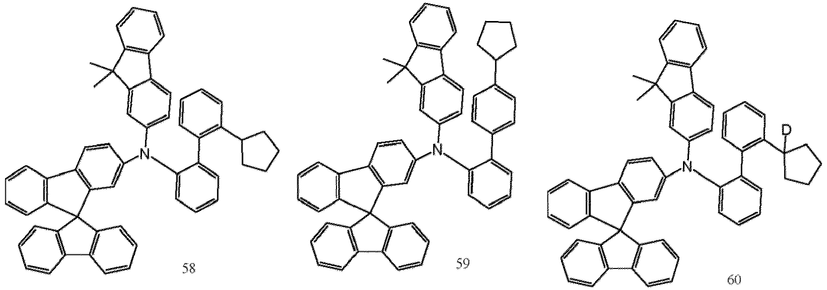

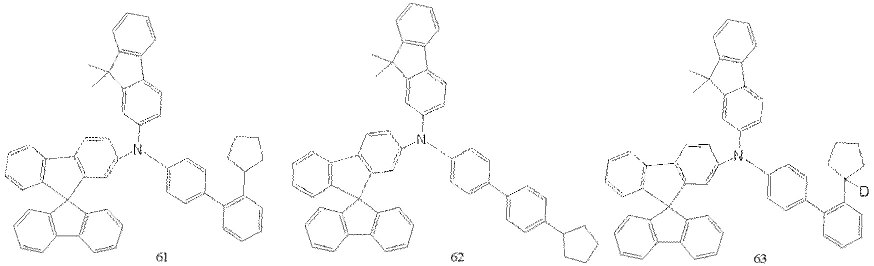

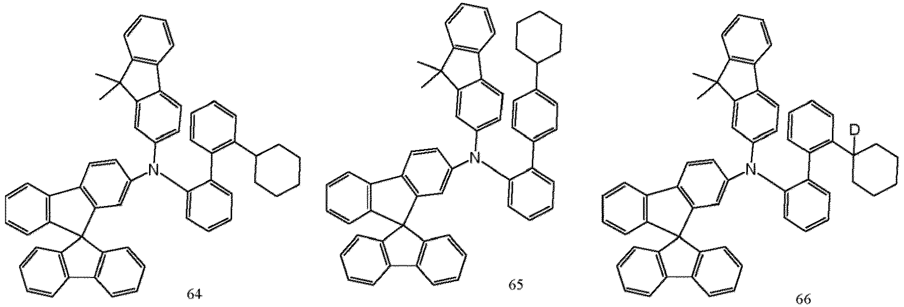

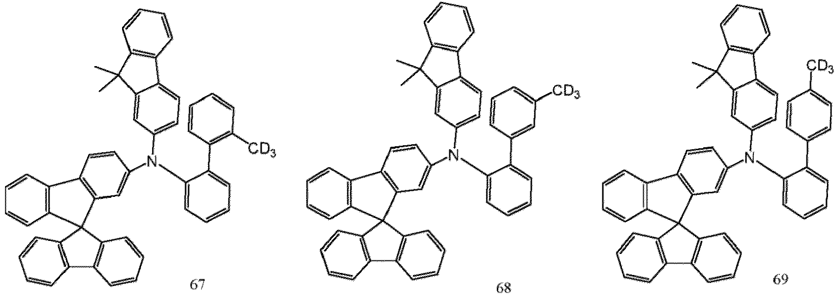

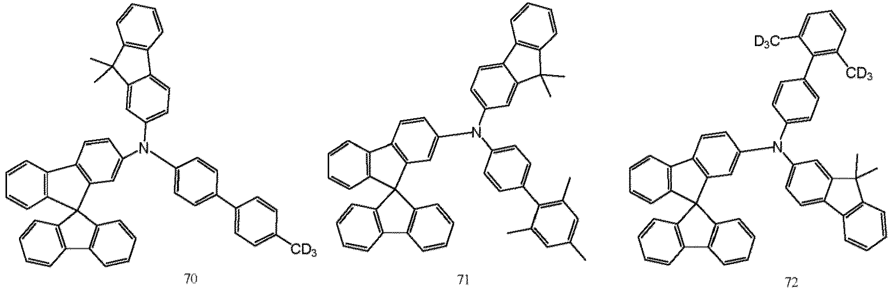

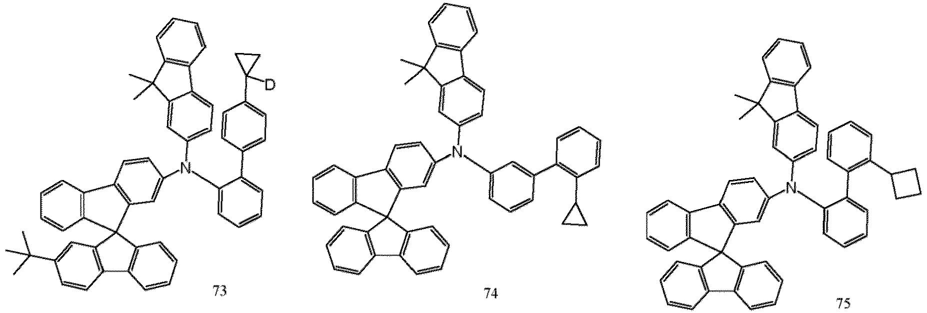

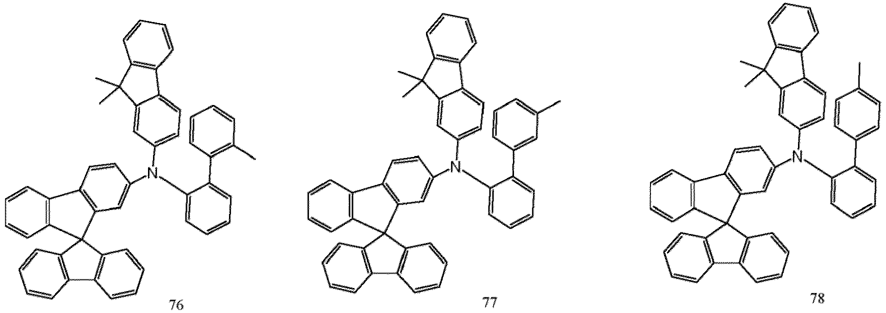

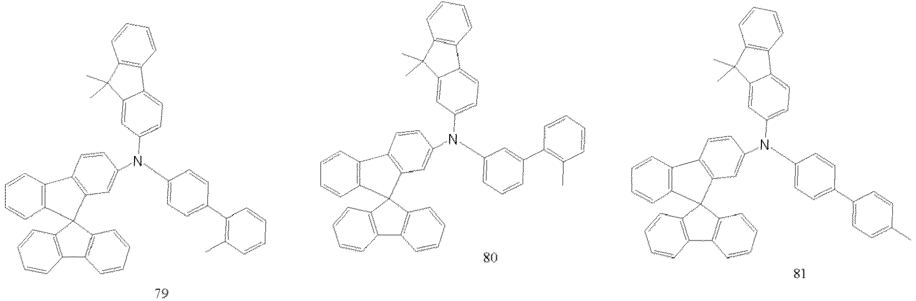

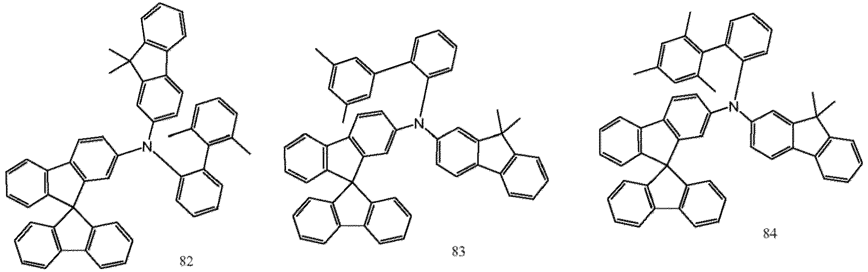

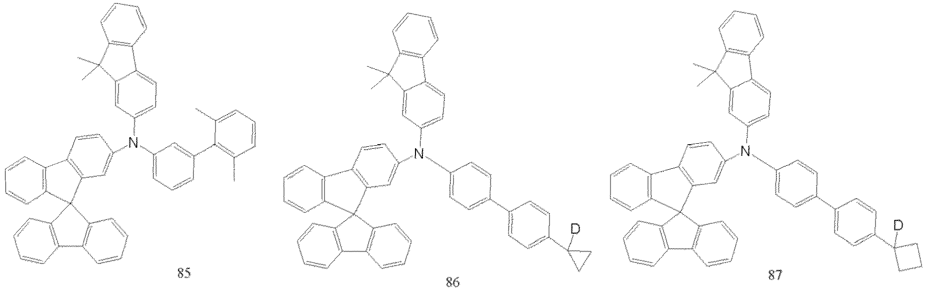

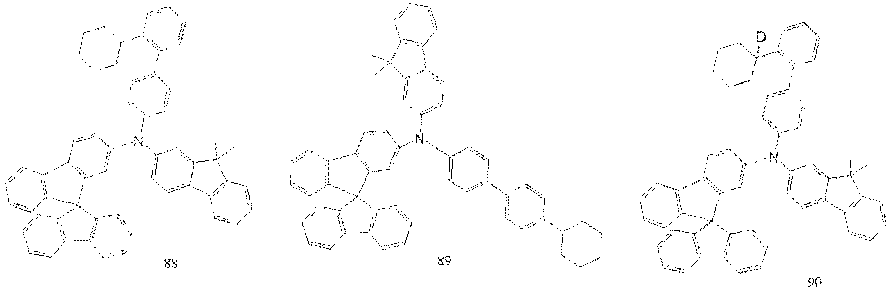

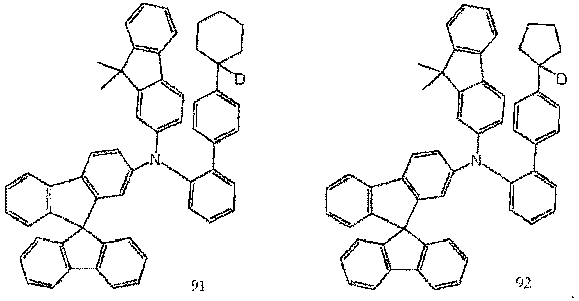

5. The organic electroluminescent device according to claim 1, wherein the hole injection layer (2) contains a compound as shown in formula A: 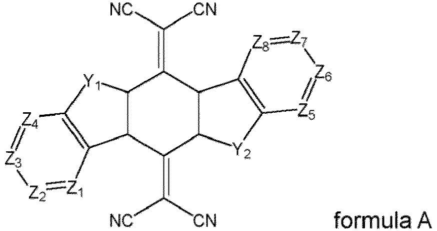
in formula A, Y₁ and Y₂ are the same or different, and are each independently O or S;
Z₁-Z₈ are each independently N, CH or CR₄;
R₄ is an alkyl of C1-C4, and at least one hydrogen in the alkyl of C1-C4 is substituted or unsubstituted with a deuterium atom or a fluorine atom;
or, the hole injection layer (2) contains compounds as shown in formula B: 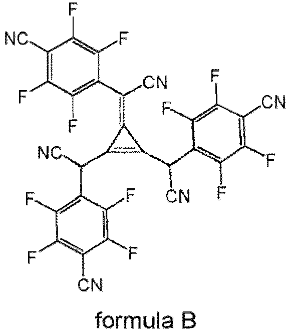
or, the hole injection layer (2) contains compounds as shown in formula C: 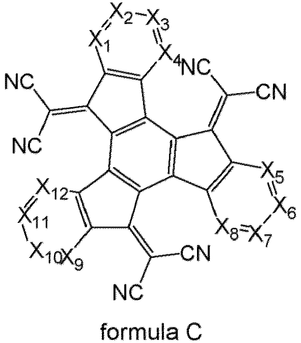
in formula C, X₁-X₁₂ are each independently selected from CR₅ or N;
R₅ selects from the group consisting of: hydrogen, deuterium, halogen, cyano, substituted or unsubstituted alkyl with 1-20 carbon atoms, substituted or unsubstituted cycloalkyl with 3-20 ring carbon atoms, substituted or unsubstituted heteroalkyl having 1-20 carbon atoms, substituted or unsubstituted arylalkyl with 7-30 carbon atoms, substituted or unsubstituted alkoxy with 1-20 carbon atoms, substituted or unsubstituted aryloxy with 6-30 carbon atoms, substituted or unsubstituted alkenyl with 2-20 carbon atoms, substituted or unsubstituted aryl with 6-30 carbon atoms, substituted or unsubstituted heteroaryl with 3-30 carbon atoms, substituted or unsubstituted alkylsilyl with 3-20 carbon atoms, substituted or unsubstituted arylsilyl with 6-20 carbon atoms, and amine, acyl, carbonyl, carboxylic, ester, nitrile, isonitrile, thio, sulfinyl, sulfonyl, and phosphonyl substituted or unsubstituted with 0-20 carbon atoms, and combinations thereof;
when more than one R₅ groups exist, R₅ groups are the same or different; and
any adjacent R₅ groups can optionally be linked to form a ring or a fused structure.

6. The organic electroluminescent device according to claim 5, wherein R₄ is methyl or trifluoromethyl; and Y₁ and Y₂ are the same, and both are O or S.

7. The organic electroluminescent device according to claim 5, wherein Z₁, Z₂, Z₇ or Z₈ is CH or CR₄, Z₄ or Z₅ is N or CH, and Z₃ or Z₆ is CR₄.

8. The organic electroluminescent device according to claim 5, wherein a compound shown in formula A is any one of following:

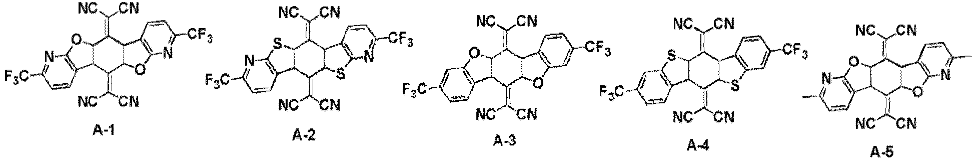

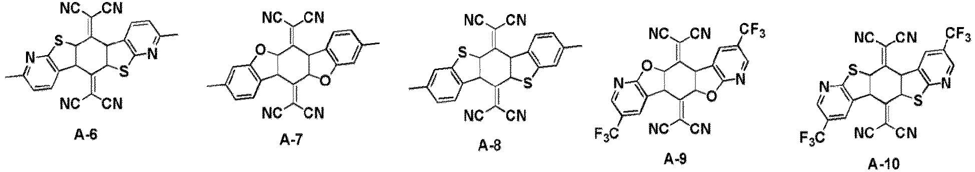

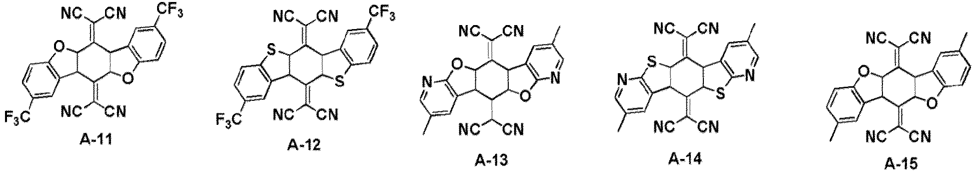

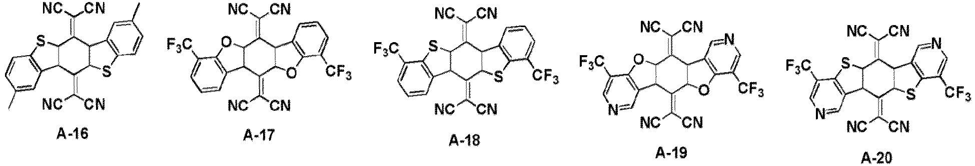

9. The organic electroluminescent device according to claim 5, wherein X₁-X₁₂ are each independently selected from CR₅ or N, R₅ is one or more of hydrogen, halogen, cyano, methyl, trifluoromethyl, methoxy, halomethoxy, or sulfur pentafluoride group.

10. The organic electroluminescent device according to claim 5, wherein a compound shown in formula C is any one of following:
